# EUROPÄISCHE PATENTSCHRIFT

(11) **EP 2 776 000 B1**
(45) Veröffentlichungstag und Bekanntmachung des Hinweises auf die Patenterteilung: **02.03.2016**
(21) Anmeldenummer: 12775500.7
(22) Anmeldetag: 19.10.2012
(51) Int. Cl.: A61K 8/49, A61Q 5/06

(54) **ZWITTERIONISCHE AZOFARBSTOFFE ZUM FÄRBEN VON KERATINHALTIGEN FASERN**
ZWITTERIONIC AZO DYESTUFFS FOR COLOURING KERATIN-CONTAINING FIBRES
COLORANTS AZOÏQUES ZWITTERIONIQUES SERVANT A COLORER DES FIBRES KÉRATINIQUES

(30) Priorität: 08.11.2011 DE 102011085906
(43) Veröffentlichungstag der Anmeldung: 17.09.2014
(73) Patentinhaber: Henkel AG & Co. KGaA, 40589 Düsseldorf (DE)
(72) Erfinder: GROß, Wibke, 41836 Hückelhoven (DE); NEMITZ, Ralph, 41363 Jüchen (DE); MOCH, Melanie, 41542 Dormagen (DE)
(86) Internationale Anmeldenummer: PCT/EP2012/070741
(87) Internationale Veröffentlichungsnummer: WO 2013/068223

(56) Entgegenhaltungen:
- EP-A1- 1 915 984
- WO-A1-2006/081245

## Beschreibung

Die vorliegende Erfindung betrifft Mittel zum Färben von keratinischen Fasern, insbesondere menschlichen Haaren, welche neuartige zwitterionische Azofarbstoffe enthalten. Weiterhin betrifft die Erfindung die Verwendung dieser neuen Azofarbstoffe in Mitteln zum Färben von Haaren sowie die Farbstoffe selbst.

Für das Färben von keratinischen Fasern kommen im allgemeinen entweder direktziehende Farbstoffe oder Oxidationsfarbstoffe zur Anwendung. Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Des Weiteren können einige Oxidationsfarbstoffvorprodukte bzw. bestimmte Mischungen von Oxidationsfarbstoffvorprodukten bei Personen mit empfindlicher Haut sensibilisierend wirken. Direktziehende Farbstoffe werden unter schonenderen Bedingungen appliziert.

Ihr Nachteil liegt jedoch darin, dass die Färbungen häufig nur über unzureichende Echtheitseigenschaften verfügen, insbesondere bei der Haarwäsche, aber auch gegenüber äußeren Einflüssen, wie Sonnenlicht oder reaktiven Umweltchemikalien, wie beispielsweise Schwimmbadwasser. Direktziehende Farbstoffe werden häufig auch zur Nuancierung von oxidativen Färbungen eingesetzt. Eine besondere Herausforderung für die Haarfärbung mit direktziehenden Farbstoffen stellt die gleichmäßige Färbung von häufig vorbehandeltem Haar, wie beispielsweise gebleichtem oder dauergewelltem Haar, dar, bei dem die Faser in den verschiedenen Längen oder verschieden behandelten Bereichen eine stark unterschiedliche Vorschädigung besitzt. Während der Färbung selbst kann das Färbemittel auf unterschiedlich vorgeschädigtem Haar ein ungleichmäßiges Färbeverhalten zeigen, aber auch durch wiederholte Haarwäschen können die Farbstoffe in den unterschiedlichen Haarbereichen verschieden stark auswaschen werden, wodurch ein uneinheitliches und damit unerwünschtes Farbergebnis erzielt wird.

Für die starke Aufhellung von dunklen Haaren wird nicht nur Wasserstoffperoxid allein, sondern eine Kombination aus Wasserstoffperoxid und Persulfaten (z.B. Ammoniumpersulfat, Kaliumpersulfat und/oder Natriumpersulfat) eingesetzt. Soll also dunkles Haar in einem Schritt stark aufgehellt und gleichzeitig in einem leuchtenden Farbton gefärbt werden, ist der Einsatz einer Mischung aus Wasserstoffperoxid, Persulfaten und einem direktziehenden Farbstoff von Vorteil. Obwohl dem Fachmann zum Färben von Haaren viele intensiv färbende direktziehende Farbstoffe bekannt sind, so kennt er doch nur eine sehr begrenzte Auswahl an Farbstoffen, die die starken oxidativen Bedingungen, wie sie eine Mischung der oben genannten Oxidationsmittel darstellt, ohne Zersetzung überstehen. Zudem weisen die aus dem Stand der Technik bekannten oxidationsstabilen Farbstoffe im Hinblick auf ihre übrigen Echtheitseigenschaften gravierende Nachteile auf.

Für das gleichzeitige Färben und starke Aufhellen von Haaren besteht damit nach wie vor ein Bedarf an Farbstoffen mit hoher Stabilität gegenüber starken Oxidationsmitteln. Auch unter diesen extremen Anwendungsbedingungen sollen diese Farbstoffe ihre positiven Echtheits- und Färbeeigenschaften nicht verlieren.

Aufgabe der vorliegenden Anmeldung ist es daher, Färbemittel für keratinische Fasern, insbesondere menschliche Haare, bereitzustellen, die hinsichtlich der Farbtiefe und der Echtheitseigenschaften, wie insbesondere Licht-, Reib- und Waschechtheit sowie Schweiß- und Kaltwellechtheit, gute anwendungstechnische Eigenschaften besitzen. Schließlich ist besonders wünschenswert, Färbemittel mit gutem Egalisiervermögen bereitzustellen. Im Falle der gleichzeitigen Anwendung mit Oxidationsfarbstoffen und/oder Oxidationsmitteln sollen die direktziehenden Farbstoffe eine ausreichende Stabilität gegen Wasserstoffperoxid und andere Oxidationsmittel besitzen und ihre positiven Echtheits- und Färbeeigenschaften nicht verlieren. Zudem sollten möglichst leuchtende und intensive Färbungen erzielt werden.

Es hat sich gezeigt, dass leuchtende und intensive Färbungen insbesondere mit kationischen direktziehenden Farbstoffen erzeugt werden können. Kationische Farbstoffe zeichnen sich oft durch eine besonders hohe Affinität zu Keratinfasern aus, was auf die Wechselwirkungen der positiven Ladungen der Farbstoffe mit negativ geladenen Strukturbestandteilen der keratinischen Fasern zurückgeführt werden kann. Daher lassen sich mit kationischen Farbstoffen oftmals besonders intensive Färbungen erzielen. Bedingt durch die in der Molekülstruktur vorhandene positive Ladung sind viele dieser Farbstoffe jedoch auch anfällig gegenüber hydrolytischen Spaltungen, was sich nachteilig auf die Lagerstabilität der Verbindungen auswirkt. Bei Lagerung in Formulierung sind viele kationische Farbstoffe daher nur mangelhaft lagerstabil. Dieser Mangel an Stabilität ist insbesondere dann ausgeprägt, wenn die Formulierungen alkalisch eingestellt sind. Es ist daher weiterhin die Aufgabe der vorliegenden Erfindung, neuartige, intensiv färbende Farbstoffe mit hoher Affinität zur Keratinfaser bereitzustellen, die auch bei längerer Lagerung in Formulierung keine Abbauprozesse durchlaufen und insbesondere eine hohe Stabilität gegenüber Alkalisierungsmitteln und alkalischen Formulierungen besitzen.

Es wurde überraschenderweise gefunden, dass sich bestimmte zwitterionische Azofarbstoffe, die sowohl eine positive Ladung innerhalb des Chromophors als auch eine über eine Linkereinheit mit dem Chromophor verbundene Schwefelsäureester-Gruppierung aufweisen, sehr gut als direktziehende Farbstoffe für die Haarfärbung eignen. In Ausfärbungen werden intensive Farbnuancen mit sehr guten Echtheitseigenschaften erhalten, insbesondere auch in Anwesenheit von Oxidationsmitteln. Bei Einarbeitung der Farbstoffe in kosmetische, alkalisch eingestellte Formulierungen weisen sie eine gute Lagerstabiltiät auf. Diese direktziehenden Farbstoffe liefern ebenfalls bei gleichzeitiger Anwendung von Oxidationsmitteln wie Wasserstoffperoxid oder einem Gemisch aus Wasserstoffperoxid und Persulfaten intensive Nuancen ohne Abschwächung von Farbintensität und Farbbrillanz. Auf diese Weise wird das gleichzeitige Aufhellen und Färben von Haaren möglich, wodurch auch auf dunklem Haar eine leuchtende Farbgebung erzielt werden kann.

Aus EP 1915984 sind Azofarbstoffe, die eine kationische, spezifisch substituierte Thiazol-Einheit enthalten, als Haarfärbemittel bekannt. Die darin genannten Farbstoffmoleküle enthalten eine Phenylazo-Einheit, die eine über eine Alkylgruppe verbrückte Schwefelsäurester-Gruppierung aufweisen kann.

Weiterhin werden in WO 2006/081245 A1 nichtoxidative Haarfärbemittel offenbart, welche zwitterionische Azofarbstoffe mit kationischer, heterozyklischer Endgruppe enthalten, wobei diese kationische heterozyklische Endgruppe einer anionischen Alkylsulfonatgruppe substituiert ist.

Färbemittel, enthaltend zwitterionischen Azofarbstoffe gemäß nachstehender Formel (I), sind bislang nicht als Haarfarbstoffe bekannt.

Ein erster Gegenstand der vorliegenden Erfindung ist ein Mittel zum Färben von keratinischen Fasern, insbesondere menschlichen Haaren, enthaltend in einem kosmetischen Träger mindestens eine Verbindung der Formel (I), 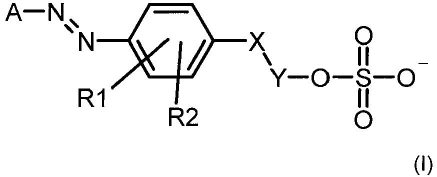 in welcher
- R1, R2: unabhängig voneinander für Wasserstoff, eine C₁-C₆-Alkylgruppe, eine C₂-C₆-Alkenylgruppe, eine Hydroxygruppe, eine C₁-C₆-Alkoxygruppe, Halogen, eine Aminogruppe, eine Acetylaminogruppe, eine Nitrogruppe oder eine Nitrilgruppe stehen,
oder aber R1 und R2 bilden, sofern sie sich in ortho-Position zueinander befinden, einen 5- oder 6-gliedrigen, gesättigten oder ungesättigten, carbozyklischen oder heterozyklischen Ring,
- X: für O oder N-R3 steht,
- R3: für Wasserstoff, eine C₁-C₆-Alkylgruppe, eine C₂-C₆-Alkenylgruppe, eine C₂-C₆-Hydroxyalkylgruppe, eine C₂-C₆-Polyhydroxyalkylgruppe, eine C₁-C₆-Cyanoalkylgruppe, eine Aryl-C₁-C₆-alkylgruppe oder eine Gruppe -Y'-O-SO₂-OM steht,
- Y, Y': jeweils unabhängig voneinander für (CH₂)ₙ oder C₂H₄-(OC₂H₄)ₙ oder (CH₂)ₙ-O-(CH₂)ₘ oder (CH₂)ₙ-N(R8)-(CH₂)ₘ stehen und n und gegebenenfalls m jeweils unabhängig voneinander für eine ganze Zahl von 1 bis 6 stehen,
- A: für eine der Strukturen (II) bis (XIII) steht, 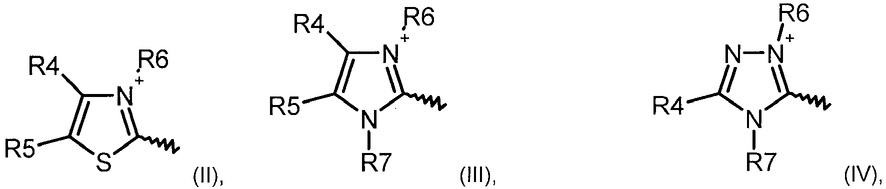 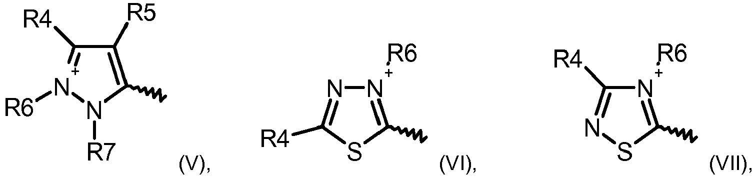 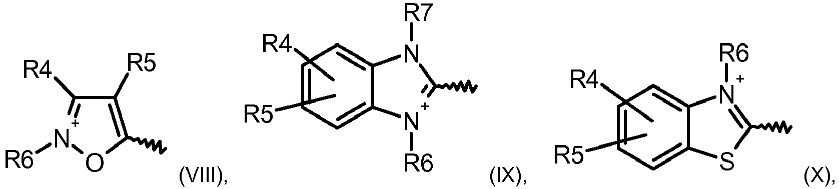 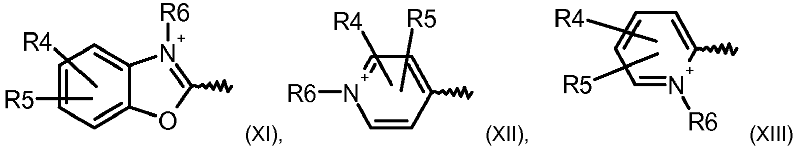 worin
R4, R5 jeweils unabhängig voneinander für Wasserstoff, eine C₁-C₆-Alkylgruppe, eine C₂-C₆-Alkenylgruppe, eine Hydroxygruppe, eine C₁-C₆-Alkoxygruppe, Halogen, eine Aminogruppe, eine Acetylaminogruppe, eine C₁-C₆-Alkylsulfonylgruppe oder eine Nitrilgruppe stehen,
oder R4 und R5 bilden, sofern sie sich in ortho-Position zueinander befinden, einen 5- oder 6-gliedrigen, gesättigten oder ungesättigten, carbozyklischen oder heterozyklischen Ring,
R6, R7 unabhängig voneinander für eine C₁-C₆-Alkylgruppe, eine C₂-C₆-Alkenylgruppe oder eine C₂-C₆-Hydroxyalkylgruppe stehen,
R8 für Wasserstoff, eine C₁-C₆-Alkylgruppe, eine C₂-C₆-Alkenylgruppe oder eine C₂-C₆-Hydroxyalkylgruppe stehen, und
M für Wasserstoff, ein Alkalimetall oder ein halbes Äquivalent eines Erdalkalimetalls steht.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.

Der erfindungsgemäß verwendete Begriff "Färben von Keratinfasern" umfasst jedwede Form der Farbveränderung der Fasern. Umfasst sind insbesondere die unter den Begriffen Tönung, Blondierung, Aufhellung, Bleiche, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise färbende Blondierungen.

Die erfindungsgemäßen Mittel enthalten die Verbindungen der Formel (I) in einem kosmetischen Träger. Dieser kosmetische Träger ist bevorzugt wässrig, alkoholisch oder wässrig-alkoholisch. Zum Zwecke der Haarbehandlung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch möglich, zur Lagerung eine pulverförmige oder auch tablettenförmige Formulierung bereitzustellen. Diese wird dann vor der Anwendung in einem wässrigen Lösungsmittel oder mit organischen Lösungsmitteln bzw. mit Gemischen aus Wasser und organischen Lösungsmitteln unter Erhalt der Anwendungsmischung vermengt. Ein wässriger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Bevorzugte erfindungsgemäße Mittel sind dadurch gekennzeichnet, dass sie zusätzlich ein nichtwässriges Lösungsmittel enthalten, wobei bevorzugte erfindungsgemäße Mittel das Lösungsmittel in einer Konzentration von 0,1 bis 30 Gew.-%, bevorzugt in einer Konzentration von 1 bis 20 Gew.-%, ganz besonders bevorzugt in einer Konzentration von 2 bis 10 Gew.-%, jeweils bezogen auf das Mittel, enthalten.

Im Folgenden werden Beispiele für die in Formel (I) genannten Substituenten R1, R2, R3, R4, R5, R6, R7 und R8 exemplarisch genannt: Beispiele für C₁-C₆-Alkylgruppen sind -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃, -(CH₂)₄CH₃, -(CH₂)₅CH₃. Besonders bevorzugte Alkylreste sind Methyl und Ethyl. Beispiele für C₂-C₆-Alkenylgruppen sind Vinyl, Prop-2-enyl (Allyl), 2-Methyl-prop-2-enyl, But-3-enyl, But-2-enyl, Pent-4-enyl oder Pent-3-enyl._Beispiele für C₂-C₆-Hydroxyalkylgruppen sind -CH₂CH₂OH, -CH₂CH₂CH₂OH, -CH₂CH(OH)CH₃, -CH₂CH₂CH₂CH₂OH, wobei die Gruppe -CH₂CH₂OH bevorzugt ist. Beispiele für C₂-C₆-Polyhydroxyalkylgruppen sind 1,2-Dihydroxyethyl, 2,3-Dihydroxypropyl, 3,4-Dihydroxybutyl und die 2,4-Dihydroxybutyl. Beispiele für C₁-C₆-Alkoxy-Gruppen sind Methoxy und Ethoxy, bevorzugt Methoxy. Beispiele für Halogen sind Fluor, Chlor, Brom und Iod, wobei Chlor und Brom bevorzugt sind. Beispiele für Cyano-C₁-C₆-alkylgruppen sind Cyanomethyl und 2-Cyanoethyl. Beispiele für Aryl-C₁-C₆-alkylgruppen sind Phenylmethyl (Benzyl), 2-Phenylethyl und 1-Phenylethyl. Die Reste R1 und R2 und/oder die Reste R4 und R5 können, sofern sie sich in ortho-Position zueinander befinden, einen 5-oder 6-gliedrigen, gesättigten oder ungesättigten, carbozyklischen oder heterozyklischen Ring bilden. Ein Beispiel für einen ungesättigten carbozyklischen Ring ist Benzen. Beispiele für gesättigte carbozyklische Ringe sind Cyclohexan und Cyclopentan. Beispiele für ungesättigte heterozyklische Ringe sind Pyridin, Pyrimidin, Pyrazin, Pyridazin, Pyrrol, Imidazol, Furan undThiophen. Beispiele für gesättigte heterozyklische Ringe sind Pyrrolidin, Piperidin, Morpholin, 1,4-Dioxan und Tetrahydrofuran. Beispiele für eine Alkylsulfonylgruppe sind Methylsulfonyl, Ethylsulfon und Propylsulfonyl.

Erfindungsgemäße Strukturen der allgemeinen Formel (I) besitzen einen Heterozyklus A, welcher ein quartäres Stickstoffatom und damit eine positive Ladung trägt. Weiterhin besitzen die Verbindungen der allgemeinen Formel (I) eine über Y an das chromophore System gebundene anionische Schwefelsäureester-Gruppierung. Damit erfolgt die Neutralisation der beiden vorhandenen Ladungen innerhalb des Moleküls, der Farbstoff liegt in zwitterionischer Form vor bildet ein inneres Salz.

Bevorzugte Reste A sind Thiazolium-Gruppen (II), Imidazolium-Gruppen (III), 1,2,4-Triazolium-Gruppen (IV), 1,3,4-Thiadiazolium-Gruppen (VI), 1,2,4-Thiadiazolium-Gruppen (VII), Benzimidazolium-Gruppen (IX), Benzothiazolium-Gruppen (X) und die Pyridinium-Gruppen (XII) und (XIII). Innerhalb dieser Gruppe der bevorzugten Reste A zeichnen sich die Gruppen (II), (III), (IV), (VII), (X), (XII) und (XIII) durch besonders gute Färbeeigenschaften aus und sind daher besonders bevorzugt. Explizit ganz besonders bevorzugt ist es, wenn der Rest A für eine der Gruppen (II), (III), (IV), (VII) oder (X) steht.

Eine weitere Ausführungsform des ersten Erfindungsgegenstands ist ein erfindungsgemäßes Mittel, welches dadurch gekennzeichnet ist, dass es mindestens eine Verbindung der Formel (I) enthält, in welcher A für eine der Strukturen (II), (III), (IV), (VI), (VII), (IX), (X), (XII), oder (XIII) steht, bevorzugt für eine der Strukturen (II), (III), (IV), (VII), (X), (XII) oder (XIII) steht, und besonders bevorzugt für eine der Strukturen (II), (III), (IV), (VII) oder (X) steht.

Weiterhin ist es bevorzugt, wenn X für eine Gruppe N-R3 steht. Y steht vorteilhafter Weise für (CH₂)ₙ mit n gleich 2 oder 3.

Eine weitere Ausführungsform des ersten Erfindungsgegenstands ist ein erfindungsgemäßes Mittel, welches dadurch gekennzeichnet ist, dass es mindestens eine Verbindung der Formel (I) enthält, in welcher X für N-R3 und Y für (CH₂)ₙ mit n gleich 2 oder 3 stehen.

Die erfindungsgemäßen Mittel können Verbindungen der Formel (I) enthalten, in welchen X für N-R3 steht. Mit R3 gleich der Gruppe -Y'-O-SO₂-OM können die Verbindungen der Formel (I) auch eine zweite Schwefelsäureester-Gruppierung enthalten. Erfindungsgemäß bevorzugte Verbindungen der Formel (I) enthalten jedoch nur eine Schwefelsäureester-Gruppierung. Bevorzugt stehen X für N-R3 und R3 für eine C₁-C₆-Alkylgruppe oder eine C₂-C₆-Alkenylgruppe, bevorzugt für eine C₁-C₆-Alkylgruppe, insbesondere bevorzugt für eine Methyl- oder eine Ethylgruppe.

Eine weitere Ausführungsform des ersten Erfindungsgegenstands ist ein erfindungsgemäßes Mittel, welches dadurch gekennzeichnet ist, dass es mindestens eine Verbindung der Formel (I) enthält, in welcher X für N-R3 und R3 für eine C₁-C₆-Alkylgruppe oder eine C₂-C₆-Alkenylgruppe, bevorzugt für eine C₁-C₆-Alkylgruppe, insbesondere bevorzugt für eine Methyl- oder eine Ethylgruppe, stehen. Weiterhin stehen die Reste R1 und R2 unabhängig voneinander bevorzugt für Wasserstoff, eine C₁-C₆-Alkylgruppe, eine C₁-C₆-Alkoxygruppe oder Halogen. Besonders bevorzugt stehen R1 und R2 unabhängig voneinander für Wasserstoff oder eine C₁-C₆-Alkylgruppe, und insbesondere bevorzugt jeweils für Wasserstoff.

Eine weitere Ausführungsform des ersten Erfindungsgegenstands ist daher ein erfindungsgemäßes Mittel, welches dadurch gekennzeichnet ist, dass es mindestens eine Verbindung der Formel (I) enthält, in welcher R1 und R2 unabhängig voneinander für Wasserstoff, eine C₁-C₆-Alkylgruppe, eine C₁-C₆-Alkoxygruppe oder Halogen stehen, bevorzugt unabhängig voneinander für Wasserstoff oder eine C₁-C₆-Alkylgruppe stehen, und insbesondere bevorzugt jeweils für Wasserstoff stehen.

Die erfindungsgemäßen Verbindungen der allgemeinen Formel (I) enthalten mit A kationische Heterozyklen, die im Ring mit den Substituenten R4 und gegebenenfalls R5 substituiert sind. Verbindungen, bei denen R4 und gegebenenfalls R5 unabhängig voneinander für Wasserstoff, eine C₁-C₆-Alkylgruppe, eine C₁-C₆-Alkoxygruppe, Halogen, eine C₁-C₆-Alkylsulfonylgruppe oder eine Nitrilgruppe stehen, zeigen im Hinblick auf die erfindungsgemäße Aufgabenstellung besondere Eignung.

Die Gruppierungen A sind kationisch und beinhalten jeweils ein quartäres Stickstoffatom, welches mit dem Rest R6 substituiert ist. Gegebenenfalls trägt ein weiteres, im Heterozyklus vorhandenes Stickstoffatom den Rest R7. Bei den Resten R6 und gegebenenfalls R7 handelt es sich in bevorzugter Weise unabhängig voneinander um eine C₁-C₆-Alkylgruppe, insbesondere bevorzugt jeweils um eine Methylgruppe.

Der Rest R8 steht bevorzugt für Wasserstoff oder eine C₁-C₆-Aklylgruppe, insbesondere bevorzugt für Wasserstoff oder eine Methylgruppe.

Erfindungsgemäß erfolgt durch M die Neutralisation der anionischen Schwefelsäureestergruppierung. Verbindungen, bei welchen M ein Proton (H⁺) oder ein Alkalimetallkation, insbesondere Natrium oder Kalium (Na⁺, K⁺) darstellt, erfüllen die erfindungsgemäße Aufgabenstellung in besonderem Maß und sind daher bevorzugt.

Erfindungsgemäß bevorzugte Mittel zum Färben von keratinischen Fasern sind dadurch gekennzeichnet, dass sie mindestens eine Verbindung der Formel (I) enthalten, die ausgewählt ist aus -2-(Methyl{4-[(3-methyl-1,3-thiazol-3-ium-2-yl)diazenyl]phenyl}amino)ethylsulfat, 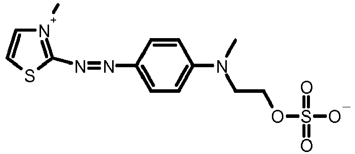
- 2-(Ethyl{4-[(3-methyl-1,3-thiazol-3-ium-2-yl)diazenyl]phenyl}amino)ethylsulfat, 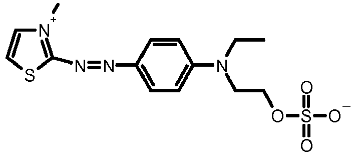
- 3-(Methyl{4-[(3-methyl-1,3-thiazol-3-ium-2-yl)diazenyl]phenyl}amino)propylsulfat, 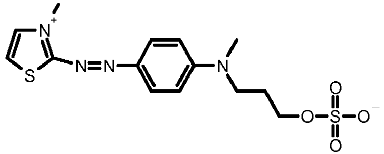
- 3-(Ethyl{4-[(3-methyl-1,3-thiazol-3-ium-2-yl)diazenyl]phenyl}amino)propylsulfat, 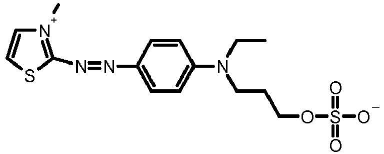
- 2-(Methyl{3-methyl-4-[(3-methyl-1,3-thiazol-3-ium-2-yl)diazenyl]phenyl}amino)-ethylsulfat, 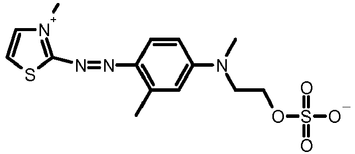
- 3-(Ethyl{3-methyl-4-[(3-methyl-1,3-thiazol-3-ium-2-yl)diazenyl]phenyl}amino)propylsulfat, 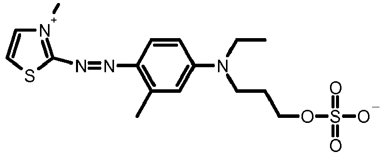
- 2-[{4-[(1,3-Dimethyl-1H-imidazol-3-ium-2-yl)diazenyl]phenyl}(methyl)amino]ethylsulfat, 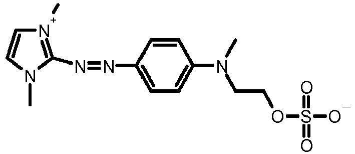
- 2-[{4-[(1,3-Dimethyl-1H-imidazol-3-ium-2-yl)diazenyl]phenyl}(ethyl)amino]ethylsulfat, 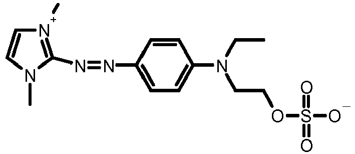
- 3-[{4-[(1,3-Dimethyl-1H-imidazol-3-ium-2-yl)diazenyl]phenyl}(ethyl)amino]propylsulfat, 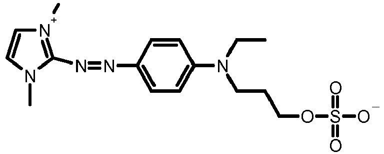
- 2-[{4-[(1,3-Dimethyl-1H-imidazol-3-ium-2-yl)diazenyl]-3-methylphenyl}(ethyl)amino]ethylsulfat, 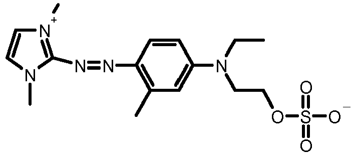
- 3-[{4-[(1,3-Dimethyl-1H-imidazol-3-ium-2-yl)diazenyl]-3-methylphenyl}(ethyl)amino]propylsulfat, 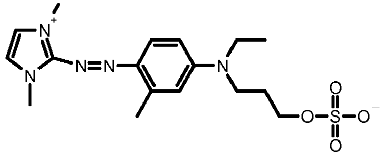
- 2-[{4-[(1,4-Dimethyl-4H-1,2,4-triazol-1-ium-5-yl)diazenyl]phenyl}(methyl)amino]ethylsulfat, 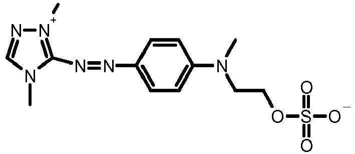
- 2-[{4-[(1,4-Dimethyl-4H-1,2,4-triazol-1-ium-5-yl)diazenyl]phenyl}(ethyl)amino]ethylsulfat, 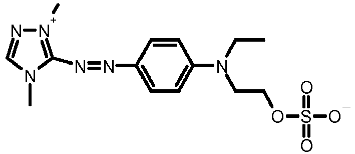
- 3-[{4-[(1,4-Dimethyl-4H-1,2,4-triazol-1-ium-5-yl)diazenyl]phenyl}(ethyl)amino]propylsulfat, 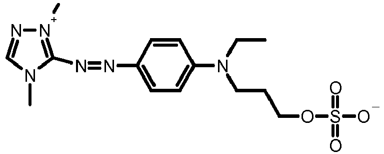
- 3-[{4-[(1,4-Dimethyl-4H-1,2,4-triazol-1-ium-5-yl)diazenyl]-3-methylphenyl}(methyl)amino]propylsulfat, 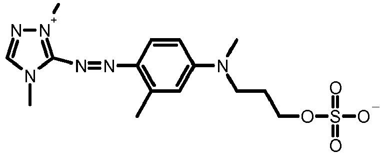
- 3-[{4-[(1,4-Dimethyl-4H-1,2,4-triazol-1-ium-5-yl)diazenyl]-3-methylphenyl}(ethyl)amino]propylsulfat, 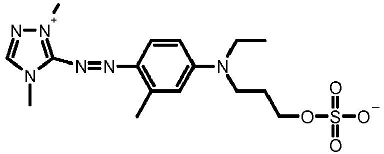
- 2-(Methyl{4-[(3-methyl-1,3,4-thiadiazol-3-ium-2-yl)diazenyl]phenyl}amino)ethylsulfat, 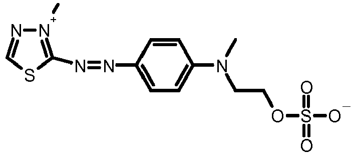
- 2-(Ethyl{4-[(3-methyl-1,3,4-thiadiazol-3-ium-2-yl)diazenyl]phenyl}amino)ethylsulfat, 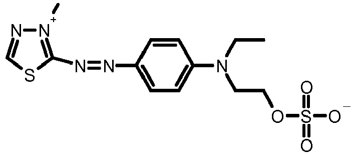
- 3-(Ethyl{4-[(3-methyl-1,3,4-thiadiazol-3-ium-2-yl)diazenyl]phenyl}amino)propylsulfat, 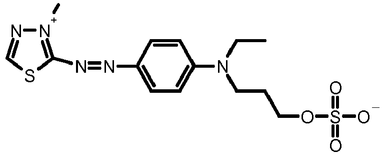
- 3-(Methyl{3-methyl-4-[(3-methyl-1,3,4-thiadiazol-3-ium-2-yl)diazenyl]phenyl}amino)propylsulfat, 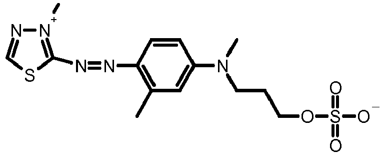
- 3-(Ethyl{3-methyl-4-[(3-methyl-1,3,4-thiadiazol-3-ium-2-yl)diazenyl]phenyl}amino)propylsulfat, 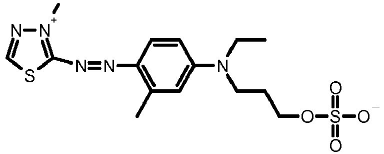
- 2-(Methyl{4-[(4-methyl-1,2,4-thiadiazol-4-ium-5-yl)diazenyl]phenyl}amino)ethylsulfat, 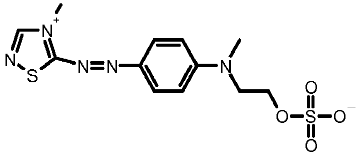
- 2-(Ethyl{4-[(4-methyl-1,2,4-thiadiazol-4-ium-5-yl)diazenyl]phenyl}amino)ethylsulfat, 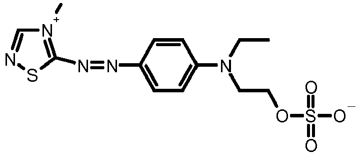
- 3-(Ethyl{4-[(4-methyl-1,2,4-thiadiazol-4-ium-5-yl)diazenyl]phenyl}amino)propylsulfat, 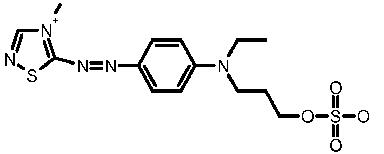
- 2-(Methyl{3-methyl-4-[(4-methyl-1,2,4-thiadiazol-4-ium-5-yl)diazenyl]phenyl}amino)ethylsulfat, 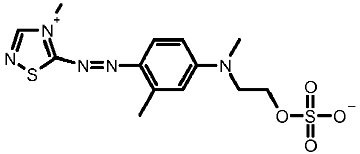
- 3-(Ethyl{3-methyl-4-[(4-methyl-1,2,4-thiadiazol-4-ium-5-yl)diazenyl]phenyl}amino)propylsulfat, 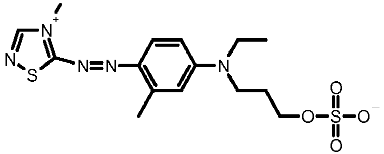
- 2-(Methyl{4-[(3-methyl-1,3-benzothiazol-3-ium-2-yl)diazenyl]phenyl}amino)ethylsulfat, 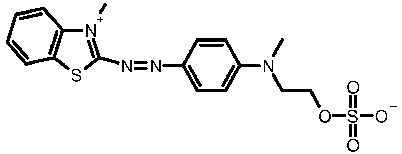
- 2-(Ethyl{4-[(3-methyl-1,3-benzothiazol-3-ium-2-yl)diazenyl]phenyl}amino)ethylsulfat, 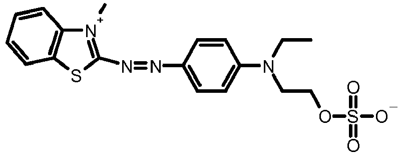
- 2-(Ethyl{4-[(3,6-dimethyl-1,3-benzothiazol-3-ium-2-yl)diazenyl]phenyl}amino)ethylsulfat, 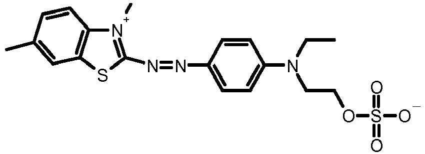
- 3-(Ethyl{4-[(3-methyl-1,3-benzothiazol-3-ium-2-yl)diazenyl]phenyl}amino)propylsulfat, 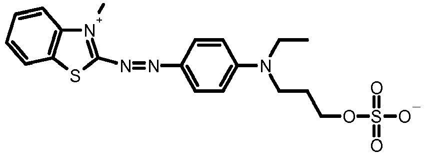
- 2-(Methyl{3-methyl-4-[(3-methyl-1,3-benzothiazol-3-ium-2-yl)diazenyl]phenyl}amino)ethylsulfat, 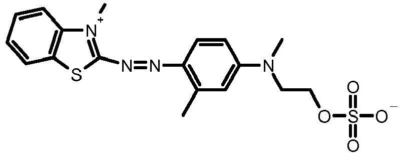
- 3-(Ethyl{3-methyl-4-[(3-methyl-1,3-benzothiazol-3-ium-2-yl)diazenyl]phenyl}amino)propylsulfat, 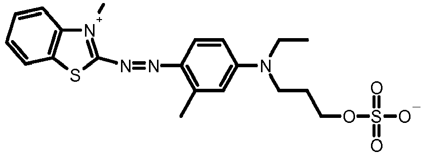
- 2-[{4-[(1,3-Dimethyl-1H-benzimidazol-3-ium-2-yl)diazenyl]phenyl}(methyl)amino]ethylsulfat, 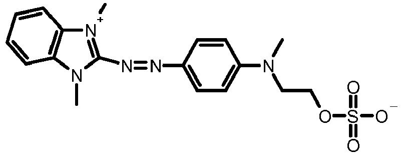
- 2-[{4-[(1,3-Dimethyl-1H-benzimidazol-3-ium-2-yl)diazenyl]phenyl}(ethyl)amino]ethylsulfat, 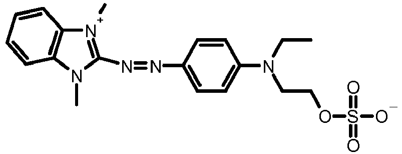
- 3-[{4-[(1,3-Dimethyl-1H-benzimidazol-3-ium-2-yl)diazenyl]phenyl}(ethyl)amino]propylsulfat, 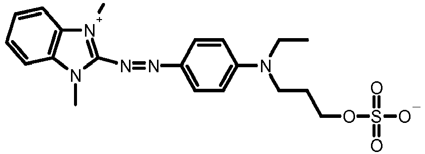
- 2-[{4-[(1,3-Dimethyl-1H-benzimidazol-3-ium-2-yl)diazenyl]-3-methylphenyl}(methyl)amino]ethylsulfat, 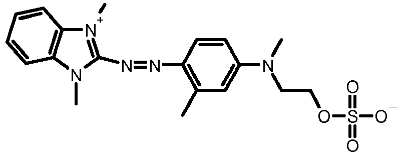
- 3-[{4-[(1,3-Dimethyl-1H-benzimidazol-3-ium-2-yl)diazenyl]-3-methylphenyl}(ethyl)amino]propylsulfat,

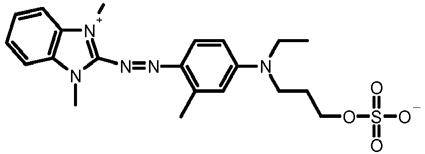

Eine explizit besonders bevorzugte Ausführungsform des ersten Erfindungsgegenstands sind Mittel, welche mindestens eine Verbindung der Formel (I) enthalten, die ausgewählt ist aus
- 2-(Methyl{4-[(3-methyl-1,3-thiazol-3-ium-2-yl)diazenyl]phenyl}amino)ethylsulfat,
- 2-(Ethyl{4-[(3-methyl-1,3-thiazol-3-ium-2-yl)diazenyl]phenyl}amino)ethylsulfat,
- 2-[{4-[(1,3-Dimethyl-1 H-imidazol-3-ium-2-yl)diazenyl]phenyl}(methyl)amino]ethylsulfat,
- 2-[{4-[(1,3-Dimethyl-1 H-imidazol-3-ium-2-yl)diazenyl]phenyl}(ethyl)amino]ethylsulfat,
- 2-[{4-[(1,4-Dimethyl-4H-1,2,4-triazol-1-ium-5-yl)diazenyl]phenyl}(methyl)amino]ethylsulfat,
- 2-[{4-[(1,4-Dimethyl-4H-1,2,4-triazol-1-ium-5-yl)diazenyl]phenyl}(ethyl)amino]ethylsulfat,
- 3-[{4-[(1,4-Dimethyl-4H-1,2,4-triazol-1-ium-5-yl)diazenyl]phenyl}(ethyl)amino]propylsulfat,
- 2-(Methyl{4-[(3-methyl-1,3,4-thiadiazol-3-ium-2-yl)diazenyl]phenyl}amino)ethylsulfat,
- 2-(Ethyl{4-[(3-methyl-1,3,4-thiadiazol-3-ium-2-yl)diazenyl]phenyl}amino)ethylsulfat,
- 2-(Methyl{4-[(4-methyl-1,2,4-thiadiazol-4-ium-5-yl)diazenyl]phenyl}amino)ethylsulfat,
- 2-(Ethyl{4-[(4-methyl-1,2,4-thiadiazol-4-ium-5-yl)diazenyl]phenyl}amino)ethylsulfat,
- 2-(Methyl{4-[(3-methyl-1,3-benzothiazol-3-ium-2-yl)diazenyl]phenyl}amino)ethylsulfat,
- 2-(Ethyl{4-[(3,6-dimethyl-1,3-benzothiazol-3-ium-2-yl)diazenyl]phenyl}amino)ethylsulfat und
- 2-(Ethyl{4-[(3-methyl-1,3-benzothiazol-3-ium-2-yl)diazenyl]phenyl}amino)ethylsulfat.

Die erfindungsgemäßen Mittel zum Färben von keratinischen Fasern enthalten die Verbindung(en) der Formel (I) vorzugsweise in Mengen oberhalb von 1 ppm und unterhalb von 10 Gew.-%, jeweils bezogen auf das gesamte Mittel. Eine bevorzugte Ausführungsform des ersten Erfindungsgegenstands ist dabei ein Mittel, welches dadurch gekennzeichnet ist, dass es die Verbindung(en) der Formel (I) jeweils in einer Menge von 0,001 bis 5 Gew.-%, bevorzugt von 0,025 bis 2,5 Gew.-%, besonders bevorzugt von 0,05 bis 2,0 Gew.-% und insbesondere bevorzugt von 0,1 bis 1,5 Gew.-%, jeweils bezogen auf das Gesamtgewicht des Mittels, enthält.

In einer weiteren bevorzugten Ausführungsform enthalten die erfindungsgemäßen Mittel neben der Verbindung der Formel (I) zusätzlich mindestens einen weiteren direktziehenden Farbstoff. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen, den Indophenolen oder den Triarylmethanen und deren physiologisch verträglichen Salzen. Die zusätzlichen direktziehenden Farbstoffe werden jeweils bevorzugt in einem Anteil von 0,001 bis 2 Gew.-%, bezogen auf das Gesamtgewicht des Mittels, eingesetzt.

Bevorzugte anionische direktziehende Farbstoffe sind die unter den internationalen Bezeichnungen bzw. Handelsnamen Bromphenolblau, Tetrabromphenolblau, Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehende Farbstoffe sind Basic Blue 7, Basic Blue 26, Basic Violet 2, Basic Violet 14, Basic Yellow 57, Basic Red 76, Basic Blue 16, Basic Blue 347 (Cationic Blue 347 / Dystar), HC Blue 16, Basic Blue 99, Basic Brown 16 und Basic Brown 17 sowie Yellow 87, Basic Orange 31 und Basic Red 51.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die fakultativ enthaltenen direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Die Färbemittel können weiterhin als aufhellende Färbemittel eingesetzt werden. Zur Erzielung des Aufhelleffekts enthalten die Mittel dazu Wasserstoffperoxid und/oder eines seiner festen Anlagerungsprodukte an organische oder anorganische Verbindungen. Beispiele für solche Anlagerungsprodukte sind an Anlagerungsprodukte an Harnstoff, Melamin sowie Natriumborat.

Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate sind Natriumpersulfat, Kaliumpersulfat und Ammoniumpersulfat.

Eine weitere Ausführungsform des ersten Erfindungsgegenstands ist daher ein Mittel zum Färben und gleichzeitigen Aufhellen von keratinischen Fasern, welches dadurch gekennzeichnet ist, dass es zusätzlich mindestens ein Oxidationsmittel, ausgewählt aus Wasserstoffperoxid, einem seiner festen Anlagerungsprodukte an organische oder anorganische Verbindungen, Natriumpersulfat, Kaliumpersulfat und Ammoniumpersulfat, enthält.

In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet.

Erfindungsgemäß bevorzugte anwendungsbereite Mittel des ersten Erfindungsgegenstands sind dadurch gekennzeichnet, dass sie 0,5 bis 15 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht des Mittels, enthalten.

Wenn die Mittel zusätzlich Persulfate enthalten, so sind diese Persulfate in dem Mittel bevorzugt in einer Menge von 1,5 bis 60 Gew.-%, vorzugsweise von 2,0 bis 45 Gew.-%, besonders bevorzugt von 2,5 bis 40 Gew.-% und insbesondere von 5 bis 30 Gew.-% , jeweils bezogen auf das Gesamtgewicht des Mittels, enthalten.

Das Mittel kann zur Verstärkung der Blondierwirkung weitere Bleichkraftverstärker enthalten, wie beispielsweise Tetraacetylethylendiamin (TAED), 1,5-Diacetyl-2,4-dioxohexahydro-1,3,5-triazin (DADHT), Tetraacetylglykoluril (TAGU), N-Nonanoylsuccinimid (NOSI), n-Nonanoyl- oder Isononanoyloxybenzolsulfonat (n- bzw. i-NOBS), Phthalsäureanhydrid, Triacetin, Ethylenglykoldiacetat und 2,5-Diacetoxy-2,5-dihydrofuran sowie Carbonatsalze bzw. Hydrogencarbonatsalze, insbesondere Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat, und stickstoffhaltige, heterocyclische Bleichkraftverstärker, wie 4-Acetyl-1-methylpyridinium-p-toluolsulfonat, 2-Acetyl-1-methylpyridinium-p-toluolsulfonat, sowie N-Methyl-3,4-dihydroisochinolinium-p-toluolsulfonat.

Zur weiteren Steigerung der Aufhellung kann der erfindungsgemäßen Zusammensetzung zusätzlich mindestens eine SiO₂-Verbindung, wie Kieselsäure oder Silicate, insbesondere Wassergläser, zugesetzt sein. Es kann erfindungsgemäß bevorzugt sein, die SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Die erfindungsgemäßen Mittel können weiterhin auch als Oxidationsfärbemittel eingesetzt werden. Solche Oxidationsfärbemittel enthalten zusätzlich mindestens ein Oxidationsfarbstoffvorprodukt, bevorzugt zumindest ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus p-Phenylendiamin, p-Toluylendiamin, 2-(2-Hydroxy-ethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1 H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin und 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind ausgewählt aus 3-Amino-phenol, 5-Amino-2-methylphenol, 3-Amino-2-chlor-6-methylphenol, 2-Hydroxy-4-aminophenoxy-ethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}-amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxy-indol, 6-Hydroxyindol, 7-Hydroxyindol, 4-Hydroxyindolin, 6-Hydroxyindolin und 7-Hydroxyindolin oder Gemischen dieser Verbindungen oder deren physiologisch verträglichen Salzen.

Die Entwicklerkomponenten und Kupplerkomponenten werden bevorzugt in einer Menge von 0,0001 bis 5,0 Gew.-%, vorzugsweise 0,001 bis 2,5 Gew.-%, jeweils bezogen auf das Gewicht des anwendungsbereiten Mittels, verwendet. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Im Falle von Oxidationsfärbemitteln enthalten die Mittel bevorzugt ein Oxidationsmittel, bevorzugt Wasserstoffperoxid. Die Mengen an Wasserstoffperoxid entsprechen den Mengen in den erfindungsgemäßen Aufhellmitteln.

Die anwendungsbereiten Färbemittel können weiterhin zusätzliche Wirk-, Hilfs- und Zusatzstoffe enthalten, um die Färbeleistung zu verbessern und weitere gewünschte Eigenschaften der Mittel einzustellen.

Vorzugsweise werden die anwendungsbereiten Färbemittel als flüssige Zubereitung bereitgestellt und den Mitteln daher zusätzlich eine oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, kationischen, zwitterionischen, amphoteren und nichtionischen Tensiden und Emulgatoren ausgewählt.

Erfindungsgemäß bevorzugte Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugte Mittel sind dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß bevorzugte Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn die Mittel weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Erfindungsgemäß geeignete Mittel können auch kationische Tenside vom Typ der quartären Ammoniumverbindungen, der Esterquats und der Amidoamine enthalten. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Eine erfindungsgemäß besonders geeignete Verbindung aus Gruppe der Amidoamine stellt das unter der Bezeichnung Tegoamid^{®} S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die kationischen Tenside sind in den erfindungsgemäß verwendeten Mitteln bevorzugt in Anteilen von 0,05 bis 10 Gew.-%, bezogen auf das gesamte Mittel, enthalten.

Die anwendungsbereiten Färbemittel können weitere Hilfs- und Zusatzstoffe enthalten. So hat es sich als vorteilhaft erwiesen, wenn die Mittel mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind anionische, synthetische Polymere; kationische, synthetische Polymere; natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen; nichtionische, synthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit. Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels zwischen 6 und 11, insbesondere zwischen 7 und 10,5, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-) Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin.

Ferner können die erfindungsgemäßen Mittel weitere Wirk-, Hilfs- und Zusatzstoffe enthalten, wie nichtionische Polymere (beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane); Silikone, wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere); kationische Polymere wie quaternisierte Celluloseether, Polysiloxane mit quaternären Gruppen wie Dimethyldiallylammoniumchlorid-Copolymere, mit Diethylsulfat quaternierte Dimethylamino-ethylmethacrylat-Vinylpyrrolidinon-Copolymere, Vinylimidazolinium-methochlorid-Copolymere; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Ein weiterer Gegenstand der Erfindung ist die Verwendung eines erfindungsgemäßen Mittels zum Färben oder zum Färben und gleichzeiten Aufhellen von keratinischen Fasern
- zur Verbesserung der Intensität und Brillanz der Färbung und/oder
- zur Verbesserung der Echtheitseigenschaften der Färbung, insbesondere der Waschechtheit, der Lichtechtheit und der Ultrablondierechtheit.

Zur Anwendung der erfindungsgemäßen Mittel eignet sich insbesondere ein Verfahren zum Färben von keratinischen Fasern, insbesondere menschlichen Haaren, welches dadurch gekennzeichnet ist, dass ein Mittel des ersten Erfindungsgegenstands auf die keratinhaltigen Fasern aufgebracht, 5 bis 60 Minuten auf der Faser belassen und anschließend mit Wasser wieder ausgespült oder mit einem Shampoo ausgewaschen wird. Bevorzugt beträgt die Einwirkzeit der anwendungsbereiten Färbemittel 5 bis 45 min, insbesondere 10 bis 40 min, besonders bevorzugt 15 bis 35 min. Während der Einwirkzeit des Mittels auf der Faser kann es vorteilhaft sein, den Aufhellvorgang durch Wärmezufuhr zu unterstützen. Die Wärmezufuhr kann durch eine externe Wärmequelle, wie z.B. warme Luft eines Warmluftgebläses, als auch, insbesondere bei einer Haaraufhellung am lebenden Probanden, durch die Körpertemperatur des Probanden erfolgen. Bei letzterer Möglichkeit wird üblicherweise die aufzuhellende Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Nach Ende der Einwirkzeit wird die verbleibende Färbezubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Aufhellmittel einen stark tensidhaltigen Träger besitzt.

Die erfindungsgemäßen Mittel können als Einkomponentenmittel (Färbe- und Aufhellmittel) oder als Mehrkomponentenmittel wie Zweikomponenten-Mittel oder Dreikomponenten-Mittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Ein Färbe- und Aufhellverfahren, bei dem die Aufhellcreme und das Oxidationsmittel zunächst getrennt vorliegen, ist dabei bevorzugt. Ein bevorzugtes erfindungsgemäßes Verfahren ist daher dadurch gekennzeichnet, dass eine Zusammensetzung auf wässriger Grundlage, enthaltend Wasserstoffperoxid, mit einem erfindungsgemäßen Mittel des ersten Erfindungsgegenstands zu einer homogenen Zusammensetzung vermischt, und diese auf das Haar aufgebracht wird.

Bezüglich weiterer bevorzugter Ausführungsformen der erfindungsgemäßen Verfahren gilt mutatis mutandis das zu den erfindungsgemäßen Mitteln Gesagte.

Die Verbindungen der allgemeinen Formel (I) eignen sich sehr gut als direktziehende Farbstoffe für die Haarfärbung. Hierbei werden Färbungen mit hoher Farbintensität und Farbbrillanz und guten Echtheitseigenschaften erhalten. Es ist auf diese Weise auch das gleichzeitige Aufhellen und Färben von Haaren möglich, wodurch auch auf dunklem Haar eine leuchtende Farbgebung erzielt werden kann.

Verbindungen der allgemeinen Formel (I) sind bislang nicht literaturbekannt. Ein weiterer Gegenstand der vorliegenden Erfindung sind daher Verbindungen der Formel (I), 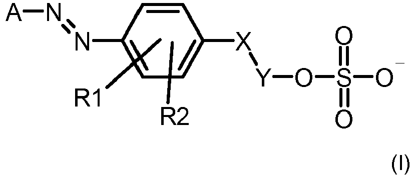 in welcher
- R1, R2: unabhängig voneinander für Wasserstoff, eine C₁-C₆-Alkylgruppe, eine C₂-C₆-Alkenylgruppe, eine Hydroxygruppe, eine C₁-C₆-Alkoxygruppe, Halogen, eine Aminogruppe, eine Acetylaminogruppe, eine Nitrogruppe oder eine Nitrilgruppe stehen,
oder aber R1 und R2 bilden, sofern sie sich in ortho-Position zueinander befinden, einen 5- oder 6-gliedrigen, gesättigten oder ungesättigten, carbozyklischen oder heterozyklischen Ring,
- X: für O oder N-R3 steht,
- R3: für Wasserstoff, eine C₁-C₆-Alkylgruppe, eine C₂-C₆-Alkenylgruppe, eine C₂-C₆-Hydroxyalkylgruppe, eine C₂-C₆-Polyhydroxyalkylgruppe, eine C₁-C₆-Cyanoalkylgruppe, eine Aryl-C₁-C₆-alkylgruppe oder eine Gruppe -Y'-O-SO₂-OM steht,
- Y, Y': jeweils unabhängig voneinander für (CH₂)ₙ oder C₂H₄-(OC₂H₄)ₙ oder (CH₂)ₙ-O-(CH₂)ₘ oder (CH₂)ₙ-N(R8)-(CH₂)ₘ stehen und n und gegebenenfalls m jeweils unabhängig voneinander für eine ganze Zahl von 1 bis 6 stehen,
- A: für eine der Strukturen (II) bis (XIII) steht, 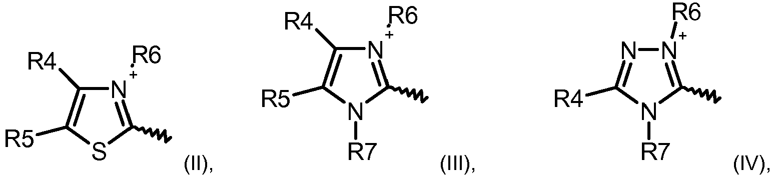 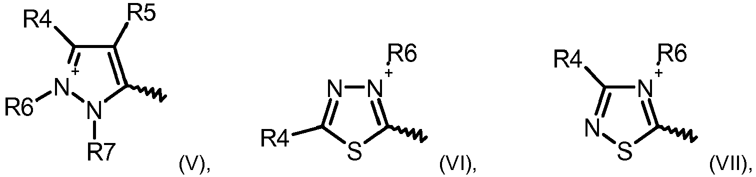 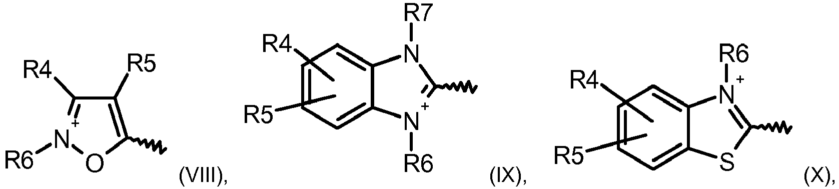 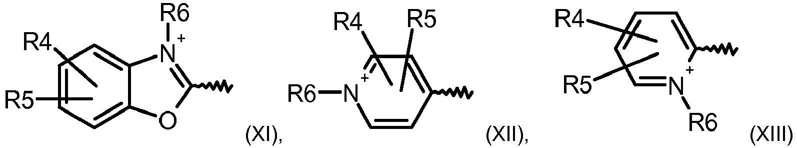
worin
- R4, R5: jeweils unabhängig voneinander für Wasserstoff, eine C₁-C₆-Alkylgruppe, eine C₂-C₆-Alkenylgruppe, eine Hydroxygruppe, eine C₁-C₆-Alkoxygruppe, Halogen, eine Aminogruppe, eine Acetylaminogruppe, eine C₁-C₆-Alkylsulfonylgruppe oder eine Nitrilgruppe stehen,
oder R4 und R5 bilden, sofern sie sich in ortho-Position zueinander befinden, einen 5- oder 6-gliedrigen, gesättigten oder ungesättigten, carbozyklischen oder heterozyklischen Ring,
- R6, R7: unabhängig voneinander für eine C₁-C₆-Alkylgruppe, eine C₂-C₆-Alkenylgruppe oder eine C₂-C₆-Hydroxyalkylgruppe stehen,
- R8: für Wasserstoff, eine C₁-C₆-Alkylgruppe, eine C₂-C₆-Alkenylgruppe oder eine C₂-C₆-Hydroxyalkylgruppe stehen, und
- M: für Wasserstoff, ein Alkalimetall oder ein halbes Äquivalent eines Erdalkalimetalls steht.

Die folgenden Beispiele sollen den Gegenstand der vorliegenden Anmeldung näher erläutern ohne ihn jedoch in irgendeiner Form zu beschränken.

### Beispiele

### 1. Synthesebeispiele

### Synthesebeispiel 1.1: Synthese von 2-(Ethyl{4-[(3-methyl-1,3-thiazol-3-ium-2-yl)diazenyl]phenyl}-amino)ethylsulfat (DZ 1)

### 1.1.1 Synthese von 2-[Ethyl(phenyl)amino]ethylhydrogensulfat

In 25,0 ml konzentrierte Schwefelsäure wurden bei einer Temperatur von 35 - 40 °C 16,5 g (0,10 mol) 2-(N-Ethylanilino)ethanol gegeben. Die Reaktion verlief leicht exotherm und wurde durch Kühlung mit Eiswasser im angegebenen Temperaturbereich gehalten. Das Gemisch wurde für wietere zwei Stunden gerührt. Nach der Beendigung der Reaktion wurde auf Eis gegossen und durch Zugabe von Wasser auf 250 ml aufgefüllt. Es resultierte eine durchsichtig gelbe Lösung, die ohne weitere Aufarbeitung in der Azokupplung eingesetzt wurde.

### 1.1.2 Synthese von 2-{Ethyl[4-(1,3-thiazol-2-yldiazenyl)phenyl]amino}ethylsulfat, Natriumsalz Diazotierung

10,0 g (0,10 mol) 2-Aminothiazol wurden in 300 ml 60 %ige, wässrige Essigsäurelösung gegeben. Nach dem Hinzufügen von 20 ml konzentrierter Schwefelsäure wurde das resultierende Gemisch auf 5 °C gekühlt. Anschließend wurden 40 ml Nitrosylschwefelsäure (40 %ig) tropfenweise hinzugefügt, unter Kühlung auf 0 °C wurde für 2 Stunden nachgerührt.

### Azokupplung

Zu der zuvor frisch hergestellten Diazoniumsalz-Lsg. wurde unter Kühlung auf 10 °C das unter 1.1.1 hergestellte Rohprodukt der Kupplungskomponente getropft. Bei Raumtemperatur wurde der Ansatz über Nacht nachgerührt. Dann wurde durch Zugabe einer 50 %igen wässrigen Natronlauge-Lösung ein pH-Wert von 7 eingestellt. Hierbei fiel ein roter Feststoff aus, welcher abfiltriert und getrocknet wurde. Zur Abtrennung von den noch vorhandenen anorganischen Salzen wurde der Feststoff unter Erwärmen mit Ethanol ausgerührt und filtriert. Anschließend wurde das Filtrat wieder komplett eingeengt.
Ausbeute: 31,4 g (88,2 %)

### 1.1.3 Synthese von 2-(Ethyl{4-[(3-methyl-1,3-thiazol-3-ium-2-yl)diazenyl]phenyl}amino)ethylsulfat (DZ 1)

30,0 g (64,2 mmol) 2-{Ethyl[4-(1,3-thiazol-2-yldiazenyl)phenyl]amino}ethylsulfat, Natriumsalz aus Stufe 1.1.2 wurden in 100 ml p-Toluolsulfonsäuremethylester für 5 Stunden bei 90 °C gerührt. Die Reaktionslösung färbte sich tief dunkelblau. Nach dem Abkühlen wurde der Ansatz mit 100 ml Toluol verdünnt. Hierbei schied sich ein dunkelblaues Produkt ab, welches von der überstehenden Lösung abgetrennt, mit Toluol gewaschen und anschließend im Vakuum getrocknet wurde. Ausbeute: 22,5 g (ca. 95 %)

### Synthesebeispiel 1.2: Synthese von 2-[{4-[(1,4-Dimethyl-4H-1,2,4-triazol-1-ium-5-yl)diazenyl]phenyl}-(ethyl)amino]ethylsulfat (DZ 2)

### 1.2.1 Synthese von 2-[Ethyl(phenyl)amino]ethylhydrogensulfat

### vgl. Beispiel 1.1.1

### 1.2.2 Synthese von 2-{Ethyl[4-(4H-1,2,4-triazol-3-yldiazenyl)phenyl]amino}ethylsulfat, Natriumsalz Diazotierung

8,4 g (0,10 mol) 3-Amino-1H-1,2,4-triazol wurden in 300 ml 60 %ige Essigsäurelösung gegeben. Nach dem Hinzufügen von 20 ml konzentrierter Schwefelsäure wurde das resultierende Gemisch auf 5 °C gekühlt. Anschließend wurden 40 ml Nitrosylschwefelsäure (40 %ig) tropfenweise hinzugefügt und für 2 Stunden unter Kühlung auf 0 °C gerührt.

### Azokupplung

Zu der zuvor frisch hergestellten Diazoniumsalz-Lsg. wurde unter Kühlung auf 10 °C das unter 1.2.1 hergestellte Rohprodukt der Kupplungskomponente getropft. Bei Raumtemperatur wurde der Ansatz über Nacht nachgerührt. Dann wurde durch Zugabe einer 50 %igen wässrigen Natronlauge-Lösung ein pH-Wert von 7 eingestellt. Hierbei fiel ein oranger Feststoff aus, welcher abfiltriert und getrocknet wurde. Zur Abtrennung von den noch vorhandenen anorganischen Salzen wurde der Feststoff unter Erwärmen mit Ethanol ausgerührt und filtriert. Anschließend wurde das Filtrat wieder komplett eingeengt.
Ausbeute: 8,8 g (26,0 %)

### 1.2.3 Synthese von 2-[{4-[(1,4-Dimethyl-4H-1,2,4-triazol-1-ium-5-yl)diazenyl]phenyl}(ethyl)amino]-ethylsulfat (DZ 2)

7,6 g (22,3 mmol) 2-{Ethyl[4-(4H-1,2,4-triazol-3-yldiazenyl)phenyl]amino}ethylsulfat, Natriumsalz aus Stufe 1.2.2. wurden in 50 ml p-Toluolsulfonsäuremethylester für 5 Stunden bei 90 °C gerührt. Die Reaktionslösung färbte sich leuchtend pink. Nach dem Abkühlen wurde der Ansatz mit 100 ml Toluol verdünnt. Hierbei schied sich das Produkt in Form eines roten Feststoffes ab, welches abfiltriert, mit Toluol gewaschen und anschließend im Vakuum getrocknet wurde. Das Produkt war mit leuchtend roter Farbe in Wasser löslich.
Ausbeute: 6,9 g (57 %)

### Synthesebeispiel 1.3: Synthese von 2-(Ethyl{4-[(3,6-dimethyl-1,3-benzothiazol-3-ium-2-yl)diazenyl]phenyl}amino)ethylsulfat (DZ 3)

### 1.3.1 Synthese von 2-[Ethyl(phenyl)amino]ethylhydrogensulfat

### vgl. Beispiel 1.1.1

### 1.3.2 Synthese von 2-{Ethyl[4-(6-methyl-1,3-benzothiazol-2-yldiazenyl)phenyl]amino}ethylsulfat, Natriumsalz

### Diazotierung

16,4 g (0,10 mol) 2-Amino-6-methylbenzothiazol wurden in 300 ml einer 60 %igen Essigsäurelösung gegeben. Nach dem Hinzufügen von 20 ml konzentrierter Schwefelsäure wurde das resultierende Gemisch auf 5 °C gekühlt. Anschließend wurden 40 ml Nitrosylschwefelsäure (40 %ig) tropfenweise hinzugefügt und für 2 Stunden unter Kühlung auf 0 °C gerührt.

### Azokupplung

Zu der zuvor frisch hergestellten Diazoniumsalz-Lsg. wurde unter Kühlung auf 10 °C das unter 1.3.1 hergestellte Rohprodukt der Kupplungskomponente getropft. Bei Raumtemperatur wurde der Ansatz über Nacht nachgerührt. Dann wurde durch Zugabe einer 50 %igen wässrigen Natronlauge-Lösung ein pH-Wert von 7eingestellt. Hierbei fiel ein violetter Feststoff aus, welcher abfiltriert und getrocknet wurde. Zur Abtrennung von den noch vorhandenen anorganischen Salzen wurde der Feststoff unter Erwärmen mit Ethanol ausgerührt und filtriert. Anschließend wurde das Filtrat wieder komplett eingeengt. Das Produkt löste sich mit leuchtend violetter Farbe in Wasser.
Ausbeute: 28,5 g (62,5 %)

### 1.3.3 Synthese von 2-(Ethyl{4-[(3,6-dimethyl-1,3-benzothiazol-3-ium-2-yl)diazenyl]phenyl}amino)-ethylsulfat (DZ 3)

23,8 g (56,6 mmol) 2-{Ethyl[4-(6-methyl-1,3-benzothiazol-2-yldiazenyl)phenyl]amino}ethylsulfat, Natriumsalz aus Stufe 1.3.2 wurden in 100 ml p-Toluolsulfonsäuremethylester für 5 Stunden bei 90 °C gerührt. Die Reaktionslösung färbte sich dunkelblau. Nach dem Abkühlen wurde der Ansatz mit 100 ml Toluol verdünnt. Hierbei schied sich das Produkt in Form einer dunkelblauen, pastösen Substanz ab, welche von der überstehenden Lösungsmittelphase abgetrennt, mehrmals mit Toluol ausgeührt und anschließend im Vakuum getrocknet wurde.
Ausbeute: 21,3 g (62,1 %)

### 2. Ausfärbungsbeispiele

### 2.1 Herstellung der Färbecremes

Es wurden die folgenden Färbecremes hergestellt:

### 2.1.1 Nichtionische Färbecreme 1

| | |
|---|---|
| Cetearyl Alcohol | 6,0 g |
| Coconut Alcohol | 6,0 g |
| PEG-40 Hydrogenated Castor Oil | 1,0 g |
| Ceteareth-12 | 3,0 g |
| Ceteareth-20 | 3,0 g |
| PHB-Methylester | 0,3 g |
| PHB-Propylester | 0,2 g |
| Phenoxyethanol | 1,0 g |
| PEG-8 | 5,0 g |
| erfindungsgemäßer DZ | 1,0 g |
| Ammoniumsulfat | 1,0 g (in 30,0 g Wasser) |
| Hydroxyethylcellulose | 1,0 g (in 15,0 g Wasser) |
| NaOH 0,1% | Ad pH-Wert |
| Wasser | ad 100 g |

Die ersten neun Komponenten wurden zusammen bei 80 °C aufgeschmolzen, danach wurde der Farbstoff zugefügt. Diese Mischung wurde mit einer Lösung des Direktziehers und des Ammoniumsulfats in 30 g Wasser emulgiert. Anschließend wurde eine Quellung von 1,0 g Natrosol 250 HR in 15,0 g Wasser hinzugegeben. Der in der Tabelle angegebene pH-Wert wurde mit 0,1% Natronlauge eingestellt, anschließend wurde mit Wasser auf 100 g aufgefüllt.

### 2.1.2 Kationische Färbecreme 2

| | |
|---|---|
| Cetearyl Alcohol | 4,0 g |
| Ceteareth-12 | 1,0 g |
| Dehyquart^{®} A-CA | 2,0 g |
| Ammoniumsulfat | 1,0 g (in 30,0 g Wasser) |
| erfindungsgemäßer DZ | 1,0 g |
| Wasser | ad 100 g |

Das Stenol 16/18 wurde zusammen mit Eumulgin B1 und Dehyquart A-CA aufgeschmolzen, danach wurde die Schmelze mit heißem Wasser emulgiert. Dann wurden der Farbstoff sowie die wässrige Ammoniumsulfatlösung hinzugegeben. Der pH-Wert wurde mit Ammoniak bzw. Zitronensäure auf den in der Tabelle angegebenen Wert eingestellt, anschließend wurde mit Wasser wurde auf 100 g aufgefüllt.

### 2.1.3 Anionische Färbecreme 3

| | |
|---|---|
| Cetearyl Alcohol | 1,0 g |
| Coconut Alcohol | 1,0 g |
| Akypo Soft^{®} RLM 45N | 1,1 g |
| PHB-Propylester | 0,1 g |
| PHB-Methylester | 0,1 g |
| Ammoniumsulfat | 1,0 g (in 30,0 g Wasser) |
| erfindungsgemäßer DZ | 1,0g g |
| Wasser | ad 100 g |

Die ersten fünf Komponenten wurden zusammen aufgeschmolzen. Diese Schmelze wurde mit heißem Wasser emulgiert, anschließend wurde der in Wasser vorgelöste bzw. vordispergierte Farbstoff hinzu gegeben sowie die Ammoniumsulfatlösung hinzugefügt. Der in der Tabelle angegebene pH-Wert wurde mit Ammoniak bzw. Zitronensäure eingestellt, danach wurde mit Wasser auf 100 g aufgefüllt.

### 2.2 Verzeichnis der eingesetzten Rohstoffe

| | |
|---|---|
| Akypo RLM 45 NV^{®} | Laurylalkohol-4.5-EO-Essigsäure-Natrium-Salz (min. 22% Aktiv-substanzgehalt; INCI-Bezeichnung: Sodium Laureth-5 Carboxylate) |
| Dehyquart^{®}A-CA | Trimethylhexadecylammoniumchlorid (ca. 24 -26 % Aktivsubstanz; INCI-Bezeichnung: Aqua (Water), Cetrimonium Chloride) |

2.3 Ausfärbungen

Jeweils 1,8 g der Färbecreme wurden auf eine ca. 6 cm lange Strähne Menschenhaares (Kerling Euronaturhaar, blond) aufgetragen und dort 30 Minuten bei 30 °C belassen. Nach Beendigung der Einwirkzeit wurde das Haar ausgespült, mit einem üblichen Haarwaschmittel gewaschen und anschließend getrocknet. Die Haarsträhnen wurden in den nachfolgend angegebenen Nuancen gefärbt.
DZ 1: 2-(Ethyl{4-[(3-methyl-1,3-thiazol-3-ium-2-yl)diazenyl]phenyl}amino)ethylsulfat
DZ 2: 2-[{4-[(1,4-Dimethyl-4H-1,2,4-triazol-1-ium-5-yl)diazenyl]phenyl}(ethyl)amino]ethylsulfat
DZ 3: 2-(Ethyl{4-[(3,6-dimethyl-1,3-benzothiazol-3-ium-2-yl)diazenyl]phenyl}amino)ethylsulfat

| Farbstoff | Färbecreme | pH-Wert | Farbnuance (Intensität) |
|---|---|---|---|
| DZ 1 | 1 | 7,0 | enzianblau (+++) |
| DZ 1 | 1 | 9,5 | schwarzblau (+++) |
| DZ 1 | 2 | 7,0 | dunkelblau (+++) |
| DZ 1 | 2 | 9,5 | schwarzblau (+++) |
| DZ 1 | 3 | 7,0 | graublau (++) |
| DZ 1 | 3 | 9,5 | dunkelblau (+++) |
| DZ 2 | 1 | 7,0 | blaurot (+++) |
| DZ 2 | 1 | 9,5 | knallrot (+++) |
| DZ 2 | 2 | 7,0 | rot (++) |
| DZ 2 | 2 | 9,5 | tiefrot (+++) |
| DZ 2 | 3 | 7,0 | blaurot (+++) |
| DZ 2 | 3 | 9,5 | knallrot (+++) |
| DZ 3 | 1 | 7,0 | kobaltblau (++) |
| DZ 3 | 1 | 9,5 | graublau (++) |
| DZ 3 | 2 | 7,0 | tiefblau (+++) |
| DZ 3 | 2 | 9,5 | tief dunkelblau (+++) |
| DZ 3 | 3 | 7,0 | lapisblau (+++) |
| DZ 3 | 3 | 9,5 | tief dunkelblau (+++) |

| | | | |
|---|---|---|---|
| Intensität: + = niedrig ++ = mittel +++ = hoch Es wurden brillante Färbungen mit besonders hoher Farbintensität erhalten. | | | |

### 3. Überprüfung der Oxidationsstabiltität der direktziehenden Farbstoffe (Ultrablondierechtheit)

### 3.1. Herstellung einer Cremeformulierung

Aus den aufgelisteten Bestandteilen wurden Cremeformulierungen wie folgt hergestellt:

| | |
|---|---|
| Cetearyl Alcohol | 1,0 g |
| Coconut Alcohol | 1,0 g |
| Akypo Soft^{®} RLM 45N | 1,1 g |
| PHB-Propylester | 0,1 g |
| PHB-Methylester | 0,1 g |
| Ammoniumsulfat | 1,0 g (in 30,0 g Wasser) |
| erfindungsgemäßer DZ | 1,0 g |
| Wasser | ad 100 g |

Die ersten fünf Komponenten wurden zusammen aufgeschmolzen. Diese Schmelze wurde mit heißem Wasser emulgiert, anschließend wurde der in Wasser vorgelöste bzw. vordispergierte Farbstoff hinzu gegeben sowie die Ammoniumsulfatlösung hinzugefügt. Der in der Tabelle angegebene pH-Wert wurde mit Ammoniak bzw. Zitronensäure eingestellt, danach wurde mit Wasser auf 100 g aufgefüllt.

### 3.2. Vermischen mit der Entwicklerdispersion

Die Cremeformulierungen wurden jeweils im Verhältnis 1:1 mit einer wie folgt zusammengesetzten Entwicklerdispersion vermischt.

| | |
|---|---|
| Natronlauge 45 % | 0,73 g |
| Dipicolinsäure | 0,10 g |
| Dinatriumpvrophosphat | 0,03 g |
| Turpinal SL (Hydroxyethan-1,1,-diphosponsäure, 1-Etidronsäure) | 1,50 g |
| Texapon NSO (INCI: Sodium laureth sulfate) | 2,00 g |
| Dow Corning DB 110 A (nicht ionische Silikonemulsion) | 0,07 g |
| Aculvn 33A (INCI: Acrylates Copolymer) | 15,00 g |
| Wasserstoffperoxid 50 % | 22,40 g |
| Wasser | ad 100 g |

### 3.3. Zugabe von Persulfat I

Anschließend wurden 100 g der unter 3.2. erhaltenen Mischung mit 8,33 g Kaliumperoxidisulfat vermischt. Der pH-Wert dieser fertigen Anwendungsmischung lag zwischen 9 und 10,2. Für den Blondier- und Färbeprozeß wurde auf Haaresträhnen Strähnen (Codes: Kerling naturweiß) von ca. 0,7 g Gewicht die 4-fache Menge der fertigen Anwendungsmischung appliziert. Nachdem die Strähnen für 30 Minuten bei 32 °C blondiert wurden, wurden sie mit einem handelsüblichen Shampoo gewaschen und mit einem Föhn getrocknet. Anschließend wurden die erhaltenen Färbungen visuell unter der Tageslichtlampe beurteilt.

| Direktziehender Farbstoff | Farbnuance (Intensität) |
|---|---|
| 2-(Ethyl{4-[(3-methyl-1,3-thiazol-3-ium-2yl)diazenyl]phenyl}amino)ethylsulfat (DZ 1) | mittelblau (+++) |
| 2-(Ethyl{4-[(3,6-dimethyl-1,3-benzothiazol-3 ium-2-yl)diazenyl]phenyl}amino) ethylsulfat (DZ 3) | dunkelblau (+++) |
| Arianor Steel Blue (Basic Blue 99) Vergleich, handelsüblicher Blaufarbstoff | blond (-) |

| | |
|---|---|
| Intensität: (-) = keine Färbung mehr sichtbar, (+) = niedrig, (++) = mittel, (+++) = hoch | |

### 3.4. Zugabe von Persulfat II

100 g der unter 3.2. erhaltenen Mischung wurden mit 20 g eines Gemisches aus Ammoniumperoxidisulfat, Natriumperoxidisuflat und Kaliumperoxidisulfat vermischt. Der pH-Wert dieser fertigen Anwendungsmischung lag zwischen 9 und 10,2. Für den Blondier- und Färbeprozeß wurde auf Haarsträhnen (Codes: Kerling naturweiß) von ca. 0,7 g Gewicht die 4-fache Menge der fertigen Anwendungsmischung appliziert. Nachdem die Strähnen für 30 Minuten bei 32 °C blondiert wurden, wurden sie mit einem handelsüblichen Shampoo gewaschen und mit einem Föhn getrocknet. Anschließend wurden die erhaltenen Färbungen visuell unter der Tageslichtlampe beurteilt.

| Direktziehender Farbstoff | Farbnuance (Intensität) |
|---|---|
| 2-(Ethyl{4-[(3-methyl-1,3-thiazol-3-ium-2-yl)diazenyl]phenyl}amino)ethylsulfat (DZ 1) | graublau (+++) |
| 2-(Ethyl{4-[(3,6-dimethyl-1,3-benzothiazol-3 ium-2-yl)diazenyl]phenyl}amino) ethylsulfat (DZ 3) | graublau (+++) |
| Arianor Steel Blue (Basic Blue 99) Vergleich, handelsüblicher Blaufarbstoff | blond (-) |

| | |
|---|---|
| Intensität: (-) = keine Färbung mehr sichtbar, (+) = niedrig, (++) = mittel, (+++) = hoch | |

## Patentansprüche

1. Mittel zum Färben von keratinischen Fasern, insbesondere menschlichen Haaren, enthaltend in einem kosmetischen Träger mindestens eine Verbindung der Formel (I), 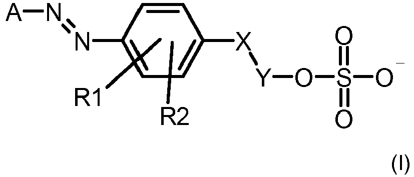 in welcher
R1, R2 unabhängig voneinander für Wasserstoff, eine C₁-C₆-Alkylgruppe, eine C₂-C₆-Alkenylgruppe, eine Hydroxygruppe, eine C₁-C₆-Alkoxygruppe, Halogen, eine Aminogruppe, eine Acetylaminogruppe, eine Nitrogruppe oder eine Nitrilgruppe stehen, oder aber R1 und R2 bilden, sofern sie sich in ortho-Position zueinander befinden, einen 5- oder 6-gliedrigen, gesättigten oder ungesättigten, carbozyklischen oder heterozyklischen Ring,
X für O oder N-R3 steht,
R3 für Wasserstoff, eine C₁-C₆-Alkylgruppe, eine C₂-C₆-Alkenylgruppe, eine C₂-C₆-Hydroxyalkylgruppe, eine C₂-C₆-Polyhydroxyalkylgruppe, eine C₁-C₆-Cyanoalkylgruppe, eine Aryl-C₁-C₆-alkylgruppe oder eine Gruppe -Y'-O-SO₂-OM steht,
Y, Y' jeweils unabhängig voneinander für (CH₂)ₙ oder C₂H₄-(OC₂H₄)ₙ oder (CH₂)ₙ-O-(CH₂)ₘ oder (CH₂)ₙ-N(R8)-(CH₂)ₘ stehen und n und gegebenenfalls m jeweils unabhängig voneinander für eine ganze Zahl von 1 bis 6 stehen,
A für eine der Strukturen (II) bis (XIII) steht, 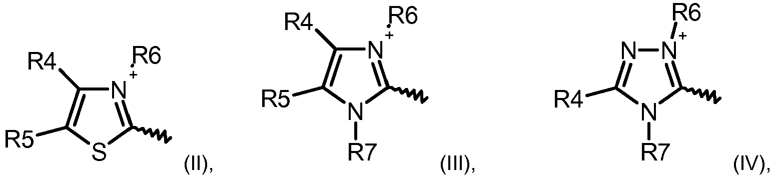 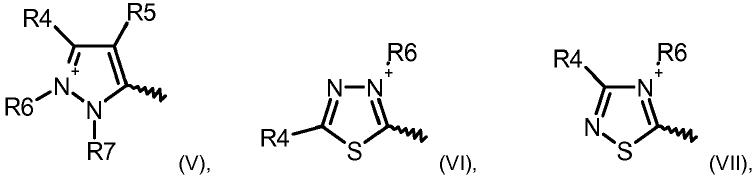 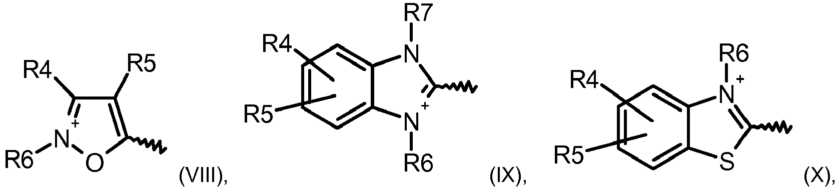 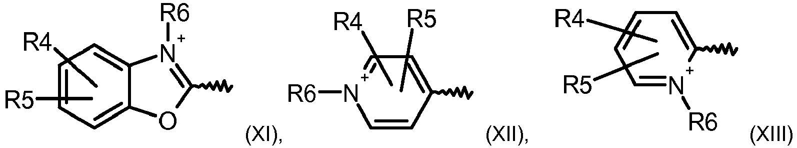 worin
R4, R5 jeweils unabhängig voneinander für Wasserstoff, eine C₁-C₆-Alkylgruppe, eine C₂-C₆-Alkenylgruppe, eine Hydroxygruppe, eine C₁-C₆-Alkoxygruppe, Halogen, eine Aminogruppe, eine Acetylaminogruppe, eine C₁-C₆-Alkylsulfonylgruppe oder eine Nitrilgruppe stehen,
oder R4 und R5 bilden, sofern sie sich in ortho-Position zueinander befinden, einen 5- oder 6-gliedrigen, gesättigten oder ungesättigten, carbozyklischen oder heterozyklischen Ring,
R6, R7 unabhängig voneinander für eine C₁-C₆-Alkylgruppe, eine C₂-C₆-Alkenylgruppe oder eine C₂-C₆-Hydroxyalkylgruppe stehen,
R8 für Wasserstoff, eine C₁-C₆-Alkylgruppe, eine C₂-C₆-Alkenylgruppe oder eine C₂-C₆-Hydroxyalkylgruppe stehen, und
M für Wasserstoff, ein Alkalimetall oder ein halbes Äquivalent eines Erdalkalimetalls steht.

2. Mittel nach Anspruch 1, **dadurch gekennzeichnet, dass** es mindestens eine Verbindung der Formel (I) enthält, in welcher A für eine der Strukturen (II), (III), (IV), (VI), (VII), (IX), (X), (XII), oder (XIII) steht, bevorzugt für eine der Strukturen (II), (III), (IV), (VII), (X), (XII) oder (XIII) steht, und besonders bevorzugt für eine der Strukturen (II), (III), (IV), (VII) oder (X) steht.

3. Mittel nach einem der Ansprüche 1 oder 2, **dadurch gekennzeichnet, dass** es mindestens eine Verbindung der Formel (I) enthält, in welcher X für N-R3 und Y für (CH₂)ₙ mit n gleich 2 oder 3 stehen.

4. Mittel nach einem der Ansprüche 1 bis 3, **dadurch gekennzeichnet, dass** es mindestens eine Verbindung der Formel (I) enthält, in welcher X für N-R3 und R3 für eine C₁-C₆-Alkylgruppe oder eine C₂-C₆-Alkenylgruppe, bevorzugt für eine C₁-C₆-Alkylgruppe, insbesondere bevorzugt für eine Methyl- oder eine Ethylgruppe, stehen.

5. Mittel nach einem der Ansprüche 1 bis 4, **dadurch gekennzeichnet, dass** es mindestens eine Verbindung der Formel (I) enthält, in welcher R1 und R2 unabhängig voneinander für Wasserstoff, eine C₁-C₆-Alkylgruppe, eine C₁-C₆-Alkoxygruppe oder Halogen stehen, bevorzugt unabhängig voneinander für Wasserstoff oder eine C₁-C₆-Alkylgruppe stehen, und insbesondere bevorzugt jeweils für Wasserstoff stehen.

6. Mittel nach einem der Ansprüche 1 bis 5, **dadurch gekennzeichnet, dass** es mindestens eine Verbindung der Formel (I) ausgewählt aus
- 2-(Methyl{4-[(3-methyl-1,3-thiazol-3-ium-2-yl)diazenyl]phenyl}amino)ethylsulfat,
- 2-(Ethyl{4-[(3-methyl-1,3-thiazol-3-ium-2-yl)diazenyl]phenyl}amino)ethylsulfat,
- 3-(Methyl{4-[(3-methyl-1,3-thiazol-3-ium-2-yl)diazenyl]phenyl}amino)propylsulfat,
- 3-(Ethyl{4-[(3-methyl-1,3-thiazol-3-ium-2-yl)diazenyl]phenyl}amino)propylsulfat,
- 2-(Methyl{3-methyl-4-[(3-methyl-1,3-thiazol-3-ium-2-yl)diazenyl]phenyl}amino)-ethylsulfat,
- 3-(Ethyl{3-methyl-4-[(3-methyl-1,3-thiazol-3-ium-2-yl)diazenyl]phenyl}amino)propylsulfat,
- 2-[{4-[(1,3-Dimethyl-1H-imidazol-3-ium-2-yl)diazenyl]phenyl}(methyl)amino]ethylsulfat,
- 2-[{4-[(1,3-Dimethyl-1H-imidazol-3-ium-2-yl)diazenyl]phenyl}(ethyl)amino]ethylsulfat,
- 3-[{4-[(1,3-Dimethyl-1H-imidazol-3-ium-2-yl)diazenyl]phenyl}(ethyl)amino]propylsulfat,
- 2-[{4-[(1,3-Dimethyl-1H-imidazol-3-ium-2-yl)diazenyl]-3-methylphenyl}(ethyl)amino]ethylsulfat,
- 3-[{4-[(1,3-Dimethyl-1H-imidazol-3-ium-2-yl)diazenyl]-3-methylphenyl}(ethyl)amino]propylsulfat,
- 2-[{4-[(1,4-Dimethyl-4H-1,2,4-triazol-1-ium-5-yl)diazenyl]phenyl}(methyl)amino]ethylsulfat,
- 2-[{4-[(1,4-Dimethyl-4H-1,2,4-triazol-1-ium-5-yl)diazenyl]phenyl}(ethyl)amino]ethylsulfat,
- 3-[{4-[(1,4-Dimethyl-4H-1,2,4-triazol-1-ium-5-yl)diazenyl]phenyl}(ethyl)amino]propylsulfat,
- 3-[{4-[(1,4-Dimethyl-4H-1,2,4-triazol-1-ium-5-yl)diazenyl]-3-methylphenyl}(methyl)amino]-propylsulfat,
- 3-[{4-[(1,4-Dimethyl-4H-1,2,4-triazol-1-ium-5-yl)diazenyl]-3-methylphenyl}(ethyl)amino]-propylsulfat,
- 2-(Methyl{4-[(3-methyl-1,3,4-thiadiazol-3-ium-2-yl)diazenyl]phenyl}amino)ethylsulfat,
- 2-(Ethyl{4-[(3-methyl-1,3,4-thiadiazol-3-ium-2-yl)diazenyl]phenyl}amino)ethylsulfat,
- 3-(Ethyl{4-[(3-methyl-1,3,4-thiadiazol-3-ium-2-yl)diazenyl]phenyl}amino)propylsulfat,
- 3-(Methyl{3-methyl-4-[(3-methyl-1,3,4-thiadiazol-3-ium-2-yl)diazenyl]phenyl}amino)propylsulfat,
- 3-(Ethyl{3-methyl-4-[(3-methyl-1,3,4-thiadiazol-3-ium-2-yl)diazenyl]phenyl}amino)propylsulfat,
- 2-(Methyl{4-[(4-methyl-1,2,4-thiadiazol-4-ium-5-yl)diazenyl]phenyl}amino)ethylsulfat,
- 2-(Ethyl{4-[(4-methyl-1,2,4-thiadiazol-4-ium-5-yl)diazenyl]phenyl}amino)ethylsulfat,
- 3-(Ethyl{4-[(4-methyl-1,2,4-thiadiazol-4-ium-5-yl)diazenyl]phenyl}amino)propylsulfat,
- 2-(Methyl{3-methyl-4-[(4-methyl-1,2,4-thiadiazol-4-ium-5-yl)diazenyl]phenyl}amino)ethylsulfat,
- 3-(Ethyl{3-methyl-4-[(4-methyl-1,2,4-thiadiazol-4-ium-5-yl)diazenyl]phenyl}amino)propylsulfat,
- 2-(Methyl{4-[(3-methyl-1,3-benzothiazol-3-ium-2-yl)diazenyl]phenyl}amino)ethylsulfat,
- 2-(Ethyl{4-[(3-methyl-1,3-benzothiazol-3-ium-2-yl)diazenyl]phenyl}amino)ethylsulfat,
- 2-(Ethyl{4-[(3,6-dimethyl-1,3-benzothiazol-3-ium-2-yl)diazenyl]phenyl}amino)ethylsulfat,
- 3-(Ethyl{4-[(3-methyl-1,3-benzothiazol-3-ium-2-yl)diazenyl]phenyl}amino)propylsulfat,
- 2-(Methyl{3-methyl-4-[(3-methyl-1,3-benzothiazol-3-ium-2-yl)diazenyl]phenyl}amino)ethylsulfat,
- 3-(Ethyl{3-methyl-4-[(3-methyl-1,3-benzothiazol-3-ium-2-yl)diazenyl]phenyl}amino)propylsulfat,
- 2-[{4-[(1,3-Dimethyl-1H-benzimidazol-3-ium-2-yl)diazenyl]phenyl}(methyl)amino]ethylsulfat,
- 2-[{4-[(1,3-Dimethyl-1H-benzimidazol-3-ium-2-yl)diazenyl]phenyl}(ethyl)amino]ethylsulfat,
- 3-[{4-[(1,3-Dimethyl-1H-benzimidazol-3-ium-2-yl)diazenyl]phenyl}(ethyl)amino]propylsulfat,
- 2-[{4-[(1,3-Dimethyl-1H-benzimidazol-3-ium-2-yl)diazenyl]-3-methylphenyl}(methyl)amino]-ethylsulfat und
- 3-[{4-[(1,3-Dimethyl-1H-benzimidazol-3-ium-2-yl)diazenyl]-3-methylphenyl}(ethyl)amino]-propylsulfat, enthält.

7. Mittel nach einem der Ansprüche 1 bis 6, **dadurch gekennzeichnet, dass** es die Verbindung(en) der Formel (I) jeweils in einer Menge von 0,001 bis 5 Gew.-%, bevorzugt von 0,025 bis 2,5 Gew.-%, besonders bevorzugt von 0,05 bis 2,0 Gew.-% und insbesondere bevorzugt von 0,1 bis 1,5 Gew.-%, jeweils bezogen auf das Gesamtgewicht des Mittels, enthält.

8. Mittel zum Färben und gleichzeitigen Aufhellen von keratinischen Fasern nach einem der Ansprüche 1 bis 7, **dadurch gekennzeichnet, dass** es zusätzlich mindestens ein Oxidationsmittel, ausgewählt aus Wasserstoffperoxid, einem seiner festen Anlagerungsprodukte an organische oder anorganische Verbindungen, Natriumpersulfat, Kaliumpersulfat und Ammoniumpersulfat, enthält.

9. Mittel zum Färben und gleichzeitigen Aufhellen von keratinischen Fasern nach einem der Ansprüche 1 bis 8, **dadurch gekennzeichnet, dass** es 0,5 bis 15 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht des Mittels, enthält.

10. Mittel zum Färben und gleichzeitigen Aufhellen von keratinischen Fasern nach einem der Ansprüche 1 bis 9, **dadurch gekennzeichnet, dass** es zusätzlich Persulfate in einer Menge von 1,5 bis 60 Gew.-%, vorzugsweise von 2,0 bis 45 Gew.-%, besonders bevorzugt von 2,5 bis 40 Gew.-% und insbesondere von 5 bis 30 Gew.-% , jeweils bezogen auf das Gesamtgewicht des Mittels, enthält.

11. Mittel zum Färben und gleichzeitigen Aufhellen von keratinischen Fasern nach einem der Ansprüche 1 bis 10, **dadurch gekennzeichnet, dass** es zusätzlich mindestens einen Komplexbildner aus der Gruppe der stickstoffhaltigen Polycarbonsäuren, insbesondere EDTA und EDDS, und Phosphonaten, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze, enthält.

12. Mittel zum Färben und gleichzeitigen Aufhellen von keratinischen Fasern nach einem der Ansprüche 1 bis 11, **dadurch gekennzeichnet, dass** es zusätzlich mindestens ein anionisches Tensid enthält.

13. Mittel zum Färben und gleichzeitigen Aufhellen von keratinischen Fasern nach einem der Ansprüche 1 bis 11, **dadurch gekennzeichnet**, das es zusätzlich mindestens ein amphoteres Tensid enthält.

14. Verwendung eines Mittels zum Färben oder zum Färben und gleichzeitigen Aufhellen von keratinischen Fasern nach einem der Ansprüche 1 bis 13
- zur Verbesserung der Intensität und Brillanz der Färbung und/oder
- zur Verbesserung der Echtheitseigenschaften der Färbung, insbesondere der Waschechtheit, der Lichtechtheit und der Ultrablondierechtheit.

15. Verbindungen der Formel (I), 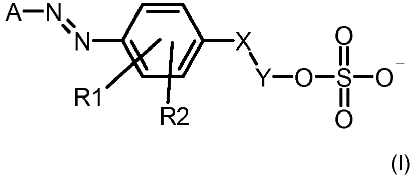 in welcher
R1, R2 unabhängig voneinander für Wasserstoff, eine C₁-C₆-Alkylgruppe, eine C₂-C₆-Alkenylgruppe, eine Hydroxygruppe, eine C₁-C₆-Alkoxygruppe, Halogen, eine Aminogruppe, eine Acetylaminogruppe, eine Nitrogruppe oder eine Nitrilgruppe stehen,
oder aber R1 und R2 bilden, sofern sie sich in ortho-Position zueinander befinden, einen 5- oder 6-gliedrigen, gesättigten oder ungesättigten, carbozyklischen oder heterozyklischen Ring,
X für O oder N-R3 steht,
R3 für Wasserstoff, eine C₁-C₆-Alkylgruppe, eine C₂-C₆-Alkenylgruppe, eine C₂-C₆-Hydroxyalkylgruppe, eine C₂-C₆-Polyhydroxyalkylgruppe, eine C₁-C₆-Cyanoalkylgruppe, eine Aryl-C₁-C₆-alkylgruppe oder eine Gruppe -Y'-O-SO₂-OM steht,
Y, Y' jeweils unabhängig voneinander für (CH₂)ₙ oder C₂H₄-(OC₂H₄)ₙ oder (CH₂)ₙ-O-(CH₂)ₘ oder (CH₂)ₙ-N(R8)-(CH₂)ₘ stehen und n und gegebenenfalls m jeweils unabhängig voneinander für eine ganze Zahl von 1 bis 6 stehen,
A für eine der Strukturen (II) bis (XIII) steht, 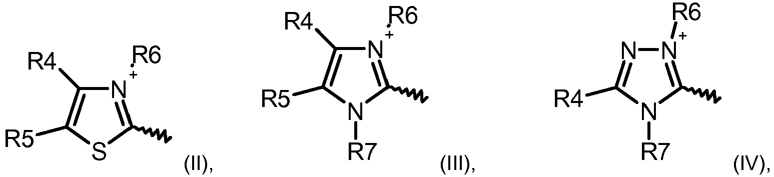 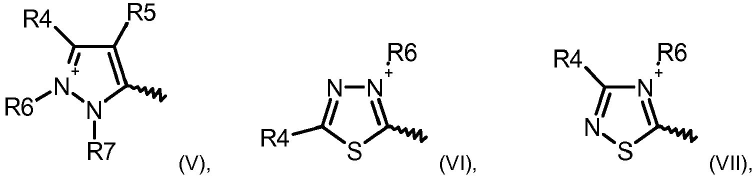 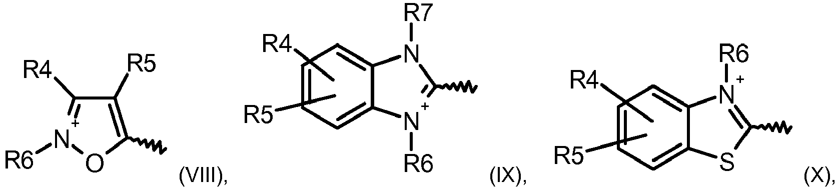 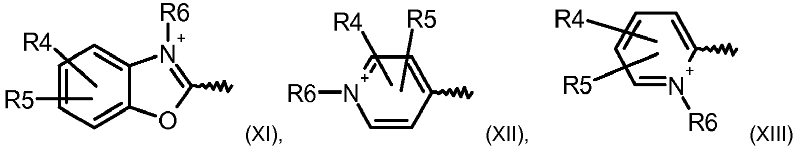 worin
R4, R5 jeweils unabhängig voneinander für Wasserstoff, eine C₁-C₆-Alkylgruppe, eine C₂-C₆-Alkenylgruppe, eine Hydroxygruppe, eine C₁-C₆-Alkoxygruppe, Halogen, eine Aminogruppe, eine Acetylaminogruppe, eine C₁-C₆-Alkylsulfonylgruppe oder eine Nitrilgruppe stehen,
oder R4 und R5 bilden, sofern sie sich in ortho-Position zueinander befinden, einen 5- oder 6-gliedrigen, gesättigten oder ungesättigten, carbozyklischen oder heterozyklischen Ring,
R6, R7 unabhängig voneinander für eine C₁-C₆-Alkylgruppe, eine C₂-C₆-Alkenylgruppe oder eine C₂-C₆-Hydroxyalkylgruppe stehen,
R8 für Wasserstoff, eine C₁-C₆-Alkylgruppe, eine C₂-C₆-Alkenylgruppe oder eine C₂-C₆-Hydroxyalkylgruppe stehen, und
M für Wasserstoff, ein Alkalimetall oder ein halbes Äquivalent eines Erdalkalimetalls steht.

## Claims

1. An agent for coloring keratinic fibers, especially human hair, containing in a cosmetic carrier at least one compound of the formula (I), 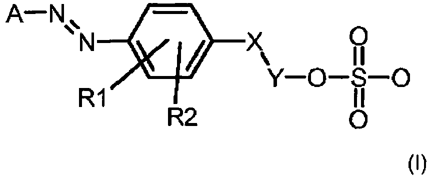 in which
R1, R2 independently of one another stand for hydrogen, a C₁-C₆ alkyl group, a C₂-C₆ alkenyl group, a hydroxy group, a C₁-C₆ alkoxy group, halogen, an amino group, an acetylamino group, a nitro group, or a nitrile group,
or, however, R1 and R2, provided they are in an ortho position to one another, form a 5- or 6-membered, saturated or unsaturated, carbocyclic, or heterocyclic ring,
X stands for O or N-R3,
R3 stands for hydrogen, a C₁-C₆ alkyl group, a C₂-C₆ alkenyl group, a C₂-C₆ hydroxyalkyl group, a C₂-C₆ polyhydroxyalkyl group, a C₁-C₆ cyanoalkyl group, an aryl-C₁-C₆-alkyl group, or a group -Y'-O-SO₂-OM,
Y, Y' in each case independently of one another stand for (CH₂)ₙ or C₂H₄-(OC₂H₄)ₙ or (CH₂)ₙ-O-(CH₂)ₘ or (CH₂)ₙ-N(R8)-(CH₂)ₘ and n and optionally m in each case independently of one another stand for an integer from 1 to 6,
A stands for one of the structures (II) to (XIII), 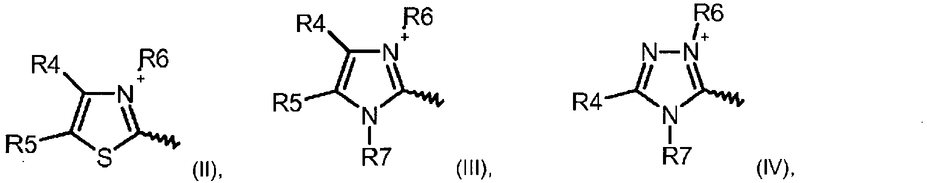 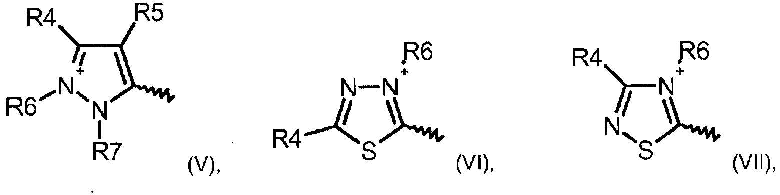 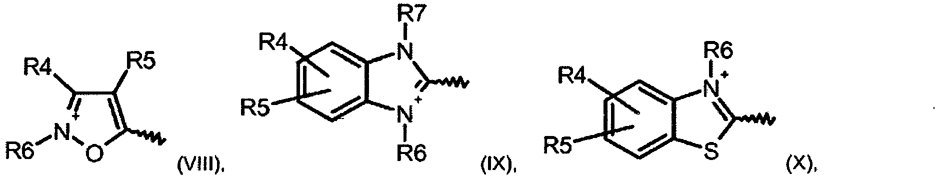 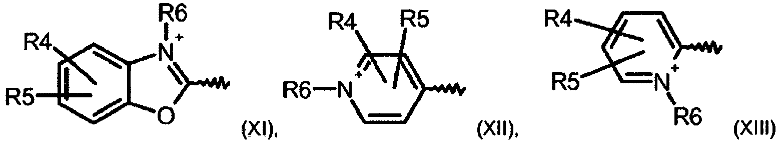 where
R4, R5 in each case independently of one another stand for hydrogen, a C₁-C₆ alkyl group, a C₂-C₆ alkenyl group, a hydroxy group, a C₁-C₆ alkoxy group, halogen, an amino group, an acetylamino group, a C₁-C₆ alkylsulfonyl group, or a nitrile group,
or R4 and R5, provided they are in an ortho position to one another, form a 5- or 6-membered, saturated or unsaturated, carbocyclic, or heterocyclic ring,
R6, R7 independently of one another stand for a C₁-C₆ alkyl group, a C₂-C₆ alkenyl group, or a C₂-C₆ hydroxyalkyl group,
R8 stands for hydrogen, a C₁-C₆ alkyl group, a C₂-C₆ alkenyl group, or a C₂-C₆ hydroxyalkyl group, and
M stands for hydrogen, an alkali metal, or half an equivalent of an alkaline earth metal.

2. The agent according to claim 1, **characterized in that** it contains at least one compound of the formula (I), in which A stands for one of the structures (II), (III), (IV), (VI), (VII), (IX), (X), (XII), or (XIII), preferably for one of the structures (II), (III), (IV), (VII), (X), (XII), or (XIII), and particularly preferably for one of the structures (II), (III), (IV), (VII), or (X).

3. The agent according to one of claims 1 or 2, **characterized in that** it contains at least one compound of the formula (I), in which X stands N-R3 and Y for (CH₂)ₙ with n being equal to 2 or 3.

4. The agent according to one of claims 1 to 3, **characterized in that** it contains at least one compound of the formula (I), in which X stands for N-R3 and R3 for a C₁-C₆ alkyl group or a C₂-C₆ alkenyl group, preferably for a C₁-C₆ alkyl group, particularly preferably for a methyl or ethyl group.

5. The agent according to one of claims 1 to 4, **characterized in that** it contains at least one compound of the formula (I), in which R1 and R2 independently of one another stand for hydrogen, a C₁-C₆ alkyl group, a C₁-C₆ alkoxy group, or halogen, preferably independently of one another for hydrogen or a C₁-C₆ alkyl group, and particularly preferably in each case for hydrogen.

6. The agent according to one of claims 1 to 5, **characterized in that** it contains at least one compound of the formula (I) selected from
- 2-(methyl{4-[(3-methyl-1,3-thiazol-3-ium-2-yl)diazenyl]phenyl}amino)ethyl sulfate,
- 2-(ethyl{4-[(3-methyl-1,3-thiazol-3-ium-2-yl)diazenyl]phenyl}amino)ethyl sulfate,
- 3-(methyl{4-[(3-methyl-1,3-thiazol-3-ium-2-yl)diazenyl]phenyl}amino)propyl sulfate,
- 3-(ethyl{4-[(3-methyl-1,3-thiazol-3-ium-2-yl)diazenyl]phenyl}amino)propyl sulfate,
- 2-(methyl{3-methyl-4-[(3-methyl-1,3-thiazol-3-ium-2-yl)diazenyl]phenyl}amino)ethyl sulfate,
- 3-(ethyl{3-methyl-4-[(3-methyl-1,3-thiazol-3-ium-2-yl)diazenyl]phenyl}amino)propyl sulfate,
- 2-[{4-[(1,3-dimethyl-1H-imidazol-3-ium-2-yl)diazenyl]phenyl}(methyl)amino]ethyl sulfate,
- 2-[{4-[(1,3-dimethyl-1H-imidazol-3-ium-2-yl)diazenyl]phenyl}(ethyl)amino]ethyl sulfate,
- 3-[{4-[(1,3-dimethyl-1H-imidazol-3-ium-2-yl)diazenyl]phenyl}(ethyl)amino]propyl sulfate,
- 2-[{4-[(1,3-dimethyl-1H-imidazol-3-ium-2-yl)diazenyl]-3-methylphenyl}(ethyl)amino]ethyl sulfate,
- 3-[{4-[(1,3-dimethyl-1H-imidazol-3-ium-2-yl)diazenyl]-3-methylphenyl}(ethyl)amino]propyl sulfate,
- 2-[{4-[(1,4-dimethyl-4H-1,2,4-triazol-1-ium-5-yl)diazenyl]phenyl}(methyl)amino]ethyl sulfate,
- 2-[{4-[(1,4-dimethyl-4H-1,2,4-triazol-1-ium-5-yl)diazenyl]phenyl}(ethyl)amino]ethyl sulfate,
- 3-[{4-[(1,4-dimethyl-4H-1,2,4-triazol-1-ium-5-yl)diazenyl]phenyl}(ethyl)amino]propyl sulfate,
- 3-[{4-[(1,4-dimethyl-4H-1,2,4-triazol-1-ium-5-yl)diazenyl]-3-methylphenyl}(methyl)amino]propyl sulfate,
- 3-[{4-[(1,4-dimethyl-4H-1,2,4-triazol-1-ium-5-yl)diazenyl]-3-methylphenyl}(ethyl)amino]propyl sulfate,
- 2-(methyl{4-[(3-methyl-1,3,4-thiadiazol-3-ium-2-yl)diazenyl]phenyl}amino)ethyl sulfate,
- 2-(ethyl{4-[(3-methyl-1,3,4-thiadiazol-3-ium-2-yl)diazenyl]phenyl}amino)ethyl sulfate,
- 3-(ethyl{4-[(3-methyl-1,3,4-thiadiazol-3-ium-2-yl)diazenyl]phenyl}amino)propyl sulfate,
- 3-(methyl{3-methyl-4-[(3-methyl-1,3,4-thiadiazol-3-ium-2-yl)diazenyl]phenyl}amino)propyl sulfate,
- 3-(ethyl{3-methyl-4-[(3-methyl-1,3,4-thiadiazol-3-ium-2-yl)diazenyl]phenyl}amino)propyl sulfate,
- 2-(methyl{4-[(4-methyl-1,2,4-thiadiazol-4-ium-5-yl)diazenyl]phenyl}amino)ethyl sulfate,
- 2-(ethyl{4-[(4-methyl-1,2,4-thiadiazol-4-ium-5-yl)diazenyl]phenyl}amino)ethyl sulfate,
- 3-(ethyl{4-[(4-methyl-1,2,4-thiadiazol-4-ium-5-yl)diazenyl]phenyl}amino)propyl sulfate,
- 2-(methyl{3-methyl-4-[(4-methyl-1,2,4-thiadiazol-4-ium-5-yl)diazenyl]phenyl}amino)ethyl sulfate,
- 3-(ethyl{3-methyl-4-[(4-methyl-1,2,4-thiadiazol-4-ium-5-yl)diazenyl]phenyl}amino)propyl sulfate,
- 2-(methyl{4-[(3-methyl-1,3-benzothiazol-3-ium-2-yl)diazenyl]phenyl}amino)ethyl sulfate,
- 2-(ethyl{4-[(3-methyl-1,3-benzothiazol-3-ium-2-yl)diazenyl]phenyl}amino)ethyl sulfate,
- 2-(ethyl{4-[(3,6-dimethyl-1,3-benzothiazol-3-ium-2-yl)diazenyl]phenyl}amino)ethyl sulfate,
- 3-(ethyl{4-[(3-methyl-1,3-benzothiazol-3-ium-2-yl)diazenyl]phenyl}amino)propyl sulfate,
- 2-(methyl{3-methyl-4-[(3-methyl-1,3-benzothiazol-3-ium-2-yl)diazenyl]phenyl}amino)ethyl sulfate,
- 3-(ethyl{3-methyl-4-[(3-methyl-1,3-benzothiazol-3-ium-2-yl)diazenyl]phenyl}amino)propyl sulfate,
- 2-[{4-[(1,3-dimethyl-1H-benzimidazol-3-ium-2-yl)diazenyl]phenyl}(methyl)amino]ethyl sulfate,
- 2-[{4-[(1,3-dimethyl-1H-benzimidazol-3-ium-2-yl)diazenyl]phenyl}(ethyl)amino]ethyl sulfate,
- 3-[{4-[(1,3-dimethyl-1H-benzimidazol-3-ium-2-yl)diazenyl]phenyl}(ethyl)amino]propyl sulfate,
- 2-[{4-[(1,3-dimethyl-1H-benzimidazol-3-ium-2-yl)diazenyl]-3-methylphenyl}(methyl)amino]ethyl sulfate, and
- 3-[{4-[(1,3-dimethyl-1H-benzimidazol-3-ium-2-yl)diazenyl]-3-methylphenyl}(ethyl)amino]propyl sulfate.

7. The agent according to one of claims 1 to 6, **characterized in that** it contains the compound(s) of the formula (I) in each case in an amount of 0.001 to 5% by weight, preferably of 0.025 to 2.5% by weight, especially preferably of 0.05 to 2.0% by weight, and particularly preferably of 0.1 to 1.5% by weight, based in each case on the total weight of the agent.

8. The agent for the coloring and simultaneous lightening of keratinic fibers according to one of claims 1 to 7, **characterized in that** it contains in addition at least one oxidizing agent, selected from hydrogen peroxide, one of its solid adducts to organic or inorganic compounds, sodium persulfate, potassium persulfate, and ammonium persulfate.

9. The agent for the coloring and simultaneous lightening of keratinic fibers according to one of claims 1 to 8, **characterized in that** it contains 0.5 to 15% by weight, preferably to 12.5% by weight, especially preferably 2.5 to 10% by weight, and particularly 3 to 6% by weight of hydrogen peroxide, based in each case on the total weight of the agent.

10. The agent for the coloring and simultaneous lightening of keratinic fibers according to one of claims 1 to 9, **characterized in that** that it contains in addition persulfates in an amount of 1.5 to 60% by weight, preferably of 2.0 to 45% by weight, especially preferably of 2.5 to 40% by weight, and particularly of 5 to 30% by weight, based in each case on the total weight of the agent.

11. The agent for the coloring and simultaneous lightening of keratinic fibers according to one of claims 1 to 10, **characterized in that** it contains in addition at least one complexing agent from the group of nitrogen-containing polycarboxylic acids, especially EDTA and EDDS, and phosphonates, especially 1-hydroxyethane-1,1-diphosphonate (HEDP), and/or ethylenediamine tetramethylene phosphonate (EDTMP), and/or diethylenetriamine pentamethylene phosphonate (DTPMP), or the sodium salts thereof.

12. The agent for the coloring and simultaneous lightening of keratinic fibers according to one of claims 1 to 11, **characterized in that** it contains in addition at least one anionic surfactant.

13. The agent for the coloring and simultaneous lightening of keratinic fibers according to one of claims 1 to 11, **characterized in that** it contains in addition at least one amphoteric surfactant.

14. Use of an agent for the coloring or for the coloring and simultaneous lightening of keratinic fibers according to one of claims 1 to 13
- to improve the intensity of brilliance of the color and/or
- to improve the fastness properties of the color, especially the washing fastness, the light fastness, and the ultra-bleaching fastness.

15. Compounds of the formula (I), 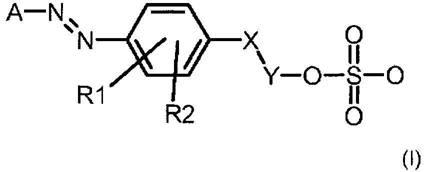 in which
R1, R2 independently of one another stand for hydrogen, a C₁-C₆ alkyl group, a C₂-C₆ alkenyl group, a hydroxy group, a C₁-C₆ alkoxy group, halogen, an amino group, an acetylamino group, a nitro group, or a nitrile group,
or, however, R1 and R2, provided they are in an ortho position to one another, form a 5- or 6-membered, saturated or unsaturated, carbocyclic, or heterocyclic ring,
X stands for O or N-R3,
R3 stands for hydrogen, a C₁-C₆ alkyl group, a C₂-C₆ alkenyl group, a C₂-C₆ hydroxyalkyl group, a C₂-C₆ polyhydroxyalkyl group, a C₁-C₆ cyanoalkyl group, an aryl-C₁-C₆-alkyl group, or a group -Y'-O-SO₂-OM,
Y, Y' in each case independently of one another stand for (CH₂)ₙ or C₂H₄-(OC₂H₄)ₙ or (CH₂)ₙ-O-(CH₂)ₘ or (CH₂)ₙ-N(R8)-(CH₂)ₘ and n and optionally m in each case independently of one another stand for an integer from 1 to 6,
A stands for one of the structures (II) to (XIII), 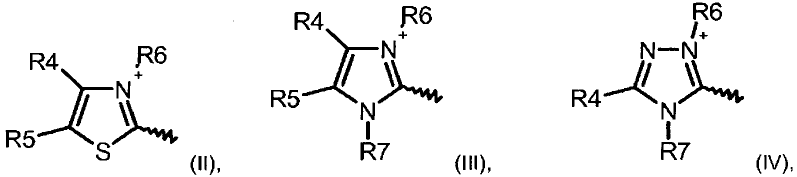 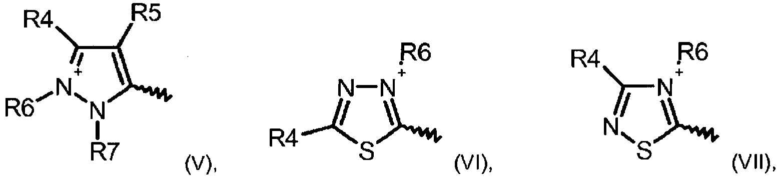 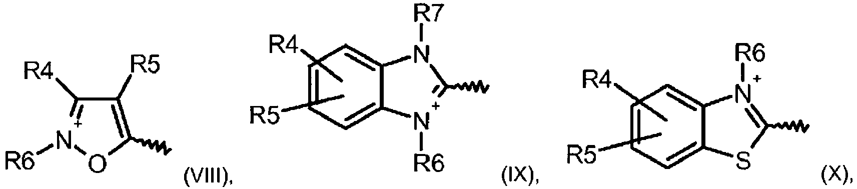 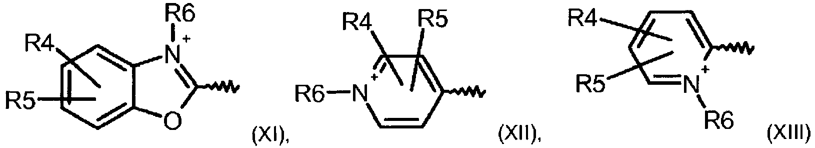 where
R4, R5 in each case independently of one another stand for hydrogen, a C₁-C₆ alkyl group, a C₂-C₆ alkenyl group, a hydroxy group, a C₁-C₆ alkoxy group, halogen, an amino group, an acetylamino group, a C₁-C₆ alkylsulfonyl group, or a nitrile group,
or R4 and R5, provided they are in an ortho position to one another, form a 5- or 6-membered, saturated or unsaturated, carbocyclic, or heterocyclic ring,
R6, R7 independently of one another stand for a C₁-C₆ alkyl group, a C₂-C₆ alkenyl group, or a C₂-C₆ hydroxyalkyl group,
R8 stands for hydrogen, a C₁-C₆ alkyl group, a C₂-C₆ alkenyl group, or a C₂-C₆ hydroxyalkyl group, and
M stands for hydrogen, an alkali metal, or half an equivalent of an alkaline earth metal.

## Revendications

1. Agent de coloration de fibres de kératine, en particulier de cheveux humains, contenant dans un support cosmétique au moins un composé de la formule (I), 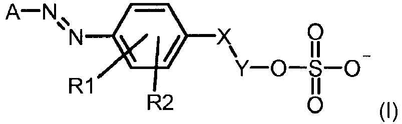 dans laquelle
R1, R2 sont indépendamment l'hydrogène, un groupe alkyle en C₁ à C₆, un groupe alcényle en C₂ à C₆, un groupe hydroxy, un groupe alcoxy en C₁ à C₆, un halogène, un groupe amino, un groupe acétylamino, un groupe nitro ou un groupe nitrile,
ou bien R1 et R2 forment, s'ils se trouvent en position ortho l'un par rapport à l'autre, un noyau carbocyclique ou hétérocyclique, saturé ou insaturé, à 5 ou 6 chaînons,
Xest O ou N-R3,
R3 est l'hydrogène, un groupe alkyle en C₁ à C₆, un groupe alcényle en C₂ à C₆, un groupe hydroxyalkyle en C₂ à C₆, un groupe polyhydroxyalkyle en C₂ à C₆, un groupe cyanoalkyle en C₁ à C₆, un groupe aryl-alkyle en C₁ à C₆, ou un groupe -Y'-O-SO₂-OM,
Y, Y' sont chacun indépendamment (CH₂)ₙ ou C₂H₄(OC₂H₄)ₙ ou (CH₂)ₙ-C)-(CH₂)ₘ ou (CH₂)ₙ-N(R8)-(CH₂)ₘ et n et éventuellement m sont chacun indépendamment un nombre entier de 1 à 6,
A représente l'une des structures (II) à (XIII), 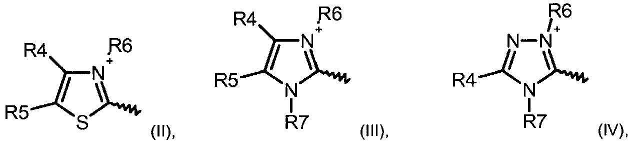 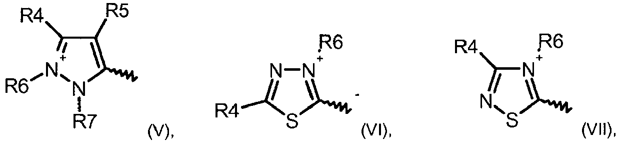 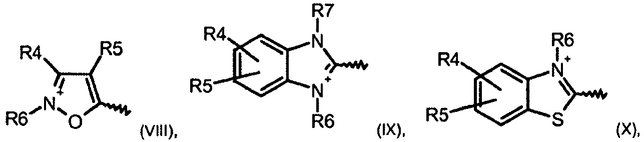 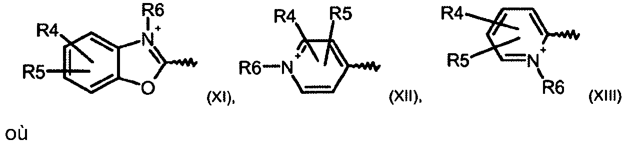
R4, R5sont chacun indépendamment l'hydrogène, un groupe alkyle en C₁ à C₆, un groupe alcényle en C₂ à C₆, un groupe hydroxy, un groupe alcoxy en C₁ à C₆, un halogène, un groupe amino, un groupe acétylamino, un groupe alkylsulfonyle en C₁ à C₆ou un groupe nitrile,
ou R4 et R5 forment, s'ils se trouvent en position ortho l'un par rapport à l'autre, un noyau carbocyclique ou hétérocyclique, saturé ou insaturé, à 5 ou 6 chaînons, R6, R7sont indépendamment un groupe alkyle en C₁ à C₆, un groupe alcényle en C₂ à C₆ou un groupe hydroxyalkyle en C₂ à C₆,
R8 est l'hydrogène, un groupe alkyle en C₁ à C₆, un groupe alcényle en C₂ à C₆ou un groupe hydroxyalkyle en C₂ à C₆, et
M est l'hydrogène, un métal alcalin ou un demi-équivalent d'un métal alcalino-terreux.

2. Agent selon la revendication 1, **caractérisé en ce qu'**il contient au moins un composé de la formule (I), dans laquelle A représente l'une des structures (II), (III), (IV), (VI), (VII), (IX), (X), (XII) ou (XIII),de préférence l'une des structures (II), (III), (IV), (VII), (X), (XII) ou (XIII), et de façon particulièrement préférée l'une des structures (II), (III), (IV), (VII) ou (X).

3. Agent selon l'une des revendications 1 ou 2, **caractérisé en ce qu'**il contient au moins un composé de la formule (I) dans laquelle X est N-R3 et Y est (CH₂)ₙ avec n égal à 2 ou 3.

4. Agent selon l'une des revendications 1 à 3, **caractérisé en ce qu'**il contient au moins un composé de la formule (I) dans laquelle X est N-R3 et R3 est un groupe alkyle en C₁ à C₆ou un groupe alcényle en C₂ à C₆, de préférence un groupe alkyle en C₁ à C₆, de manière particulièrement préférée un groupe méthyle ou éthyle.

5. Agent selon l'une des revendications 1 à 4, **caractérisé en ce qu'**il contient au moins un composé de la formule (I) dans laquelle R1 et R2 sont indépendamment l'hydrogène, un groupe alkyle en C₁ à C₆, un groupe alcoxy en C₁ à C₆ou un halogène, de préférence indépendamment l'hydrogène ou un groupe alkyle en C₁ à C₆, et de manière particulièrement préférée chacun l'hydrogène.

6. Agent selon l'une des revendications 1 à 5, **caractérisée en ce qu'**il contient au moins un composé de la formule (I) sélectionné parmi
- le sulfate de 2-(méthyl{4-[(3-méthyl-1,3-thiazol-3-ium-2-yl)diazényl]phényl}amino)éthyle,
- le sulfate de 2-(éthyl{4-[(3-méthyl-1,3-thiazol-3-ium-2-yl)diazényl]phényl}amino)éthyle,
- le sulfate de 3-(méthyl{4-[(3-méthyl-1,3-thiazol-3-ium-2-yl)diazényl]phényl}amino)propyle,
- le sulfate de 3-(éthyl{4-[(3-méthyl-1,3-thiazol-3-ium-2-yl)diazényl]phényl}amino)propyle,
- le sulfate de 2-(méthyl{3-méthyl-4-[(3-méthyl-1,3-thiazol-3-ium-2-yl)diazényl]phényl}amino)-éthyle,
- le sulfate de 3-(éthyl{3-méthyl-4-[(3-méthyl-1,3-thiazol-3-ium-2-yl)diazényl]phényl}amino)propyle,
- le sulfate de 2-[{4-[(1,3-diméthyl-1H-imidazol-3-ium-2-yl)diazényl]phényl}(méthyl)amino]éthyle,
- le sulfate de 2-[{4-[(1,3-diméthyl-1H-imidazol-3-ium-2-yl)diazényl]phényl}(éthyl)amino]éthyle,
- le sulfate de 3-[{4-[(1,3-diméthyl-1H-imidazol-3-ium-2-yl)diazényl]phényl}(éthyl)amino]propyle,
- le sulfate de 2-[{4-[(1,3-diméthyl-1H-imidazol-3-ium-2-yl)diazényl]-3-méthylphényl}(éthyl)amino]éthyle,
- le sulfate de 3-[{4-[(1,3-diméthyl-1H-imidazol-3-ium-2-yl)diazényl]-3-méthylphényl}(éthyl)amino]propyle,
- le sulfate de 2-[{4-[(1,4-diméthyl-4H-1,2,4-triazol-1-ium-5-yl)diazényl]phényl}(méthyl)amino]éthyle,
- le sulfate de 2-[{4-[(1,4-diméthyl-4H-1,2,4-triazol-1-ium-5-yl)diazényl]phényl}(éthyl)amino]éthyle,
- le sulfate de 3-[{4-[(1,4-diméthyl-4H-1,2,4-triazol-1-ium-5-yl)diazényl]phényl}(éthyl)amino]propyle,
- le sulfate de 3-[{4-[(1,4-diméthyl-4H-1,2,4-triazol-1-ium-5-yl)diazényl]-3-méthylphényl}(méthyl)amino]-propyle,
- le sulfate 3-[{4-[(1,4-diméthyl-4H-1,2,4-triazol-1-ium-5-yl)diazényl]-3-méthylphényl}(éthyl)amino]-propyle,
- le sulfate de 2-(méthyl{4-[(3-méthyl-1,3,4-thiadiazol-3-ium-2-yl)diazényl]phényl}amino)éthyle,
- le sulfate de 2-(éthyl{4-[(3-méthyl-1,3,4-thiadiazol-3-ium-2-yl)diazényl]phényl}amino)éthyle,
- le sulfate de 3-(éthyl{4-[(3-méthyl-1,3,4-thiadiazol-3-ium-2-yl)diazényl]phényl}amino)propyle,
- le sulfate de 3-(méthyl{3-méthyl-4-[(3-méthyl-1,3,4-thiadiazol-3-ium-2-yl)diazényl]phényl}amino)propyle,
- le sulfate de 3-(éthyl{3-méthyl-4-[(3-méthyl-1,3,4-thiadiazol-3-ium-2-yl)diazényl]phényl}amino)propyle,
- le sulfate de 2-(méthyl{4-[(4-méthyl-1,2,4-thiadiazol-4-ium-5-yl)diazényl]phényl}amino)éthyle,
- le sulfate de 2-(éthyl{4-[(4-méthyl-1,2,4-thiadiazol-4-ium-5-yl)diazényl]phényl}amino)éthyle,
- le sulfate de 3-(éthyl{4-[(4-méthyl-1,2,4-thiadiazol-4-ium-5-yl)diazényl]phényl}amino)propyle,
- le sulfate de 2-(méthyl{3-méthyl-4-[(4-méthyl-1,2,4-thiadiazol-4-ium-5-yl)diazényl]phényl}amino)éthyle,
- le sulfate de 3-(éthyl{3-méthyl-4-[(4-méthyl-1,2,4-thiadiazol-4-ium-5-yl)diazényl]phényl}ami
- le sulfate de 2-(méthyl{4-[(3-méthyl-1,3-benzothiazol-3-ium-2-yl)diazényl]phényl}amino)éthyle,
- le sulfate de 2-(éthyl{4-[(3-méthyl-1,3-benzothiazol-3-ium-2-yl)diazényl]phényl}amino)éthyle,
- le sulfate de 2-(éthyl{4-[(3,6-diméthyl-1,3-benzothiazol-3-ium-2-yl)diazényl]phényl}amino)éthyle,
- le sulfate de 3-(éthyl{4-[(3-méthyl-1,3-benzothiazol-3-ium-2-yl)diazényl]phényl}amino)propyle,
- le sulfate de 2-(méthyl{3-méthyl-4-[(3-méthyl-1,3-benzothiazol-3-ium-2-yl)diazényl]phényl}amino)éthyle,
- le sulfate de 3-(éthyl{3-méthyl-4-[(3-méthyl-1,3-benzothiazol-3-ium-2-yl)diazényl]phényl}amino)propyle,
- le sulfate de 2-[{4-[(1,3-diméthyl-1H-benzimidazol-3-ium-2-yl)diazényl]phényl}(méthyl)amino]éthyle,
- le sulfate de 2-[{4-[(1,3-diméthyl-1H-benzimidazol-3-ium-2-yl)diazényl]phényl}(éthyl)amino]éthyle,
- le sulfate de 3-[{4-[(1,3-diméthyl-1H-benzimidazol-3-ium-2-yl)diazényl]phényl}(éthyl)amino]propyle,
- le sulfate de 2-[{4-[(1,3-diméthyl-1H-benzimidazol-3-ium-2-yl)diazényl]-3-méthylphényl}(méthyl)amino]-éthyleet
- le sulfate de 3-[{4-[(1,3-diméthyl-1H-benzimidazol-3-ium-2-yl)diazényl]-3-méthylphényl}(éthyl)amino]-propyle.

7. Agent selon l'une des revendications 1 à 6, **caractérisé en ce que** le composé ou les composés de la formule (I) contiennent chacun dans une quantité de 0,001 à 5% en poids, de préférence de 0,025à 2,5% en poids,de manière particulièrement préférée de 0,05 à 2,0% en poids et de manière tout particulièrement préférée de 0,1 à 1,5% en poids, à chaque fois par rapport au poids total de l'agent.

8. Agent de coloration et d'éclaircissement simultanés de fibres de kératine selon l'une des revendications 1 à 7, **caractérisé en ce qu'**il contient en outre au moins un agent oxydant sélectionné parmi le peroxyde d'hydrogène, l'un de ses produits d'addition solides à des composés organiques ou minéraux, le persulfate de sodium, le persulfate de potassium et le persulfate d'ammonium.

9. Agent de coloration et d'éclaircissement simultanésde fibres de kératine selon l'une des revendications 1 à 8, **caractérisé en ce qu'**il contient de 0,5 à 15% en poids, de préférence de 1 à 12,5% en poids, de manière particulièrement préférée de 2,5 à 10% en poids, et notamment de 3 à 6% en poids de peroxyde d'hydrogène, à chaque fois par rapport au poids total de l'agent.

10. Agent de coloration et d'éclaircissement simultanés de fibres de kératine selon l'une des revendications 1 à 9, **caractérisé en ce qu'**il contient en outre des persulfates dans une quantité de 1, 5 à 60% en poids, de préférence de 2,0 à 45% en poids, de manière particulièrement préférée de 2,5 à 40% en poids et notamment de 5 à 30% en poids, à chaque fois par rapport au poids total de l'agent.

11. Agent de coloration et d'éclaircissement simultanés de fibres de kératine selon l'une des revendications 1 à 10, **caractérisé en ce qu'**il contient en outre au moins un agent complexant du groupe des acides polycarboxyliques contenant de l'azote, en particulier de l'EDTA et de l'EDDS, et des phosphonates, en particulier le 1-hydroxyéthane-1,1-diphosphonate (HEDP) et/ou l'éthylène-diamine-tétraméthylène-phosphonate (EDTMP) et/ou diéthylène-triamine-pentaméthylène-phosphonate (DTPMP) et leurs sels de sodium.

12. Agent de coloration et d'éclaircissement simultanés de fibres de kératine selon l'une des revendications 1 à 11, **caractérisé en ce qu'**il contient en outre au moins un tensioactif anionique.

13. Agent de coloration et d'éclaircissement simultanés de fibre de kératine selon l'une des revendications 1 à 11, **caractérisé en ce qu'**il contient en outre au moins un tensioactif amphotère.

14. Utilisation d'un agent de coloration ou de coloration et d'éclaircissement simultanés de fibres de kératine selon l'une des revendications 1 à 13,
- pour améliorer l'intensité et l'éclat de la couleur et/ou
- pour améliorer les propriétés de résistance de la coloration, en particulier la résistance au lavage, la résistance à la lumière et la résistance à la décoloration excessive.

15. Composés de la formule (I), 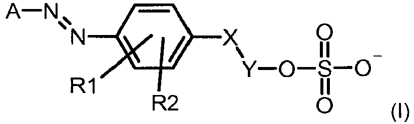 dans laquelle
R1, R2 sont indépendamment l'hydrogène, un groupe alkyle en C₁ à C₆, un groupe alcényle en C₂ à C₆, un groupe hydroxy, un groupe alcoxy en C₁ à C₆, un halogène, un groupe amino, un groupe acétylamino, un groupe nitro ou un groupe nitrile,
ou bien R1 et R2 forment, s'ils se trouvent en position ortho l'un par rapport à l'autre, un noyau carbocyclique ou hétérocyclique, saturé ou insaturé, à 5 ou 6 chaînons,
X est O ou N-R3,
R3 est l'hydrogène, un groupe alkyle en C₁ à C₆, un groupe alcényle en C₂ à C₆, un groupe hydroxyalkyle en C₂ à C₆, un groupe polyhydroxyalkyle en C₂ à C₆, un groupe cyanoalkyle en C₁ à C₆, un groupe aryl-alkyle en C₁ à C₆, ou un groupe -Y'-O-SO₂-OM,
Y, Y' sont chacun indépendamment (CH₂)ₙ ou C₂H₄(OC₂H₄)ₙ ou (CH₂)ₙ-O-(CH₂)ₘ ou (CH₂)ₙ-N(R8)-(CH₂)ₘ et n et éventuellement m sont chacun indépendamment un nombre entier de 1 à 6,
A représente l'une des structures (II) à (XIII), 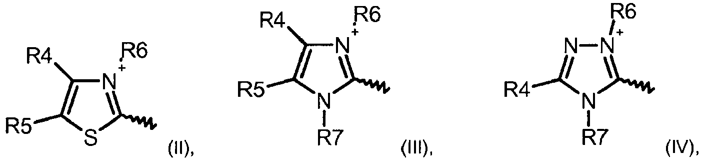 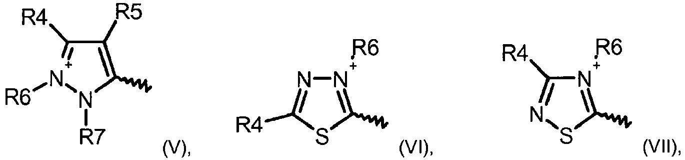 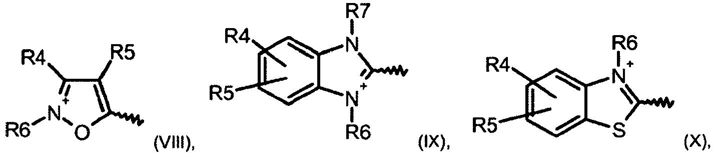 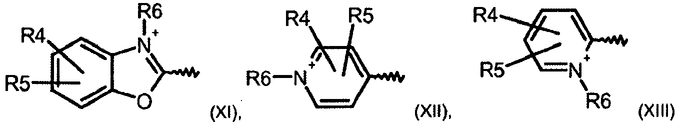 où
R4, R5 sont chacun indépendamment l'hydrogène, un groupe alkyle en C₁ à C₆, un groupe alcényle en C₂ à C₆, un groupe hydroxy, un groupe alcoxy en C₁ à C₆, un halogène, un groupe amino, un groupe acétylamino, un groupe alkylsulfonyle en C₁ à C₆ ou un groupe nitrile,
ou R4 et R5 forment, s'ils se trouvent en position ortho l'un par rapport à l'autre, un noyau carbocyclique ou hétérocyclique, saturé ou insaturé, à 5 ou 6 chaînons, R6, R7 sont indépendamment un groupe alkyle en C₁ à C₆, un groupe alcényle en C₂ à C₆ ou un groupe hydroxyalkyle en C₂ à C₆,
R8 est l'hydrogène, un groupe alkyle en C₁ à C₆, un groupe alcényle en C₂ à C₆ ou un groupe hydroxyalkyle en C₂ à C₆, et
M est l'hydrogène, un métal alcalin ou un demi-équivalent d'un métal alcalino-terreux.
